# EUROPÄISCHE PATENTSCHRIFT

(11) **EP 4 663 148 B1**
(45) Veröffentlichungstag und Bekanntmachung des Hinweises auf die Patenterteilung: **05.08.2026**
(21) Anmeldenummer: 25182591.5
(22) Anmeldetag: 13.06.2025
(51) Int. Cl.: A61B 42/40, A61F 15/00, A61B 50/30

(54) **STERILGUT-AUSGABEVORRICHTUNG**
STERILE PRODUCT DISPENSING DEVICE
DISPOSITIF DE DISTRIBUTION DE PRODUITS STÉRILES

(30) Priorität: 14.06.2024 LU 507480
(43) Veröffentlichungstag der Anmeldung: 17.12.2025
(73) Patentinhaber: Kohler, Marius, 88214 Ravensburg (DE)
(72) Erfinder: Kohler, Marius, 88214 Ravensburg (DE)
(74) Vertreter: Gruner, Leopold Joachim

(56) Entgegenhaltungen:
- US-A1- 2006 243 635
- US-A1- 2019 240 089
- US-A1- 2020 069 405
- US-A1- 2020 086 063

## Beschreibung

### TECHNISCHES GEBIET

Die vorliegende Erfindung betrifft eine Ausgabevorrichtung zur sterilen Lagerung und Ausgabe eines Sterilguts.

### STAND DER TECHNIK

Sterile Güter, wie etwa Einmalspritzen, werden zur Wahrung der Sterilität in Verpackungen gelagert, die von einem Nutzer aufgerissen und entsorgt werden müssen. Dabei fallen erhebliche Mengen an Verpackungsmüll an. Alternativ wird medizinisches Material sowie sterile Güter in Aufbewahrungsbehältern gelagert, aus denen das medizinische Material, bzw. das Sterilgut entnommen werden kann. Das Öffnen der Verpackungen der Sterilgüter, bspw. der Verpackung von Einmalspritzen erfolgt mit der Peel-Off-Technik, bei der der Aufreißfalz zwischen Papier und Folie aufgetrennt wird. Dieser Vorgang ist zeitaufwendig und wird oft nicht korrekt ausgeführt, weshalb viele Anwender die Einmalspritze einfach durch das Papier hindurchdrücken, was mit einer erhöhten Wahrscheinlichkeit einer Kontamination der Einmalspritze einhergeht.

Aus dem Stand der Technik sind verschiedene Aufbewahrungsbehälter, die zur Abgabe eines Guts oder Sterilguts eingerichtet sind, bekannt.

Eine solche Vorrichtung ist beispielsweise bekannt aus der US 20200086063 A1**,** die ein Austauschgerät für Spritzen beschreibt. Die Vorrichtung ist derart ausgestaltet, dass sie benutzte Spritzen aufnehmen kann und unbenutzte sterile Spritzen ausgibt, sobald die benutzte Spritze wieder eingelegt ist und nachfolgend durch eine unbenutzte Spritze ausgetauscht werden soll.

Die CN 112165962 A offenbart einen Aufbewahrungsbehälter zur sukzessiven Abgabe von medizinischen Behältnissen, die bspw. befüllte Spritzen enthalten. Die Spritzen werden hierbei in medizinischen Behältnissen steril gelagert. Das Innere des Aufbewahrungsbehälters ist so konzipiert, dass ein medizinischer Behälter in die Ausgabeöffnung nachrutscht, wenn ein vorheriger medizinischer Behälter entnommen wird.

Aus der DE 29510234 U1 ist ein Behälter bekannt, der zur staubsicheren Aufbewahrung und zur Abgabe von medizinischem Sterilgut eingerichtet ist. Der Behälter umfasst eine Schublade, die teilweise aus dem Behälter gezogen werden kann, um das darin gelagerte Sterilgut zu entnehmen.

Aus der ES 2367660 T3 ist ein Behälter bekannt, der zur Lagerung und Abgabe von medizinischen Instrumenten, wie etwa Spritzen, eingerichtet ist. Gleichzeitig können benutzte medizinische Instrumente, wie etwa Spritzen vom Behälter aufgenommen werden, indem diese oben eingegeben werden. Unbenutzte medizinische Instrumente werden aus einer Ausgabeöffnung an der Unterseite entnommen. Das Innere des Behälters ist durch einen Absperrschlitten zweigeteilt. Auf der oberen Seite des Absperrschlittens werden die benutzen medizinischen Instrumente gelagert, wohingegen die unbenutzten medizinischen Instrumente im unteren Bereich des Behälters gelagert werden.

Eine ähnliche Vorrichtung ist in der US 6685017 B2 beschrieben, welche einen Behälter zur Lagerung von unbenutzten sowie benutzten Spritzen beschreibt. Letztere werden verpackt aus einer unteren Ausgabeöffnung entnommen, wohingegen benutzte Spritzen an der Oberseite in einen drehbar gelagerten Deckel eingelegt werden. Dieser wird nach dem Einlegen einer benutzen Spritze gedreht, so dass diese in den Behälter fallen kann.

In der IL 268826 A ist ebenfalls eine Vorrichtung zum Lagern und Ausgeben von sterilen Watteröllchen beschrieben. Diese werden über ein sich drehendes Rad aus dem Behälter ausgegeben und können aus dem Rad entnommen werden. Das Rad selbst wird über einen Motor gedreht, welcher von einer Elektronik angesteuert wird. Ein Annäherungssensor detektiert die Annäherung der Hand eines Nutzers, wodurch der Motor aktiviert wird, welcher das Rad einen Schritt weiterbewegt, um ein neues Wattestäbchen freizugeben.

Die US 20200385199 A1 offenbart eine weitere Vorrichtung zur Lagerung und Ausgabe steriler Güter. Hierbei sind die sterilen Güter jeweils einzeln innerhalb einer Verpackung aufbewahrt, wobei die Verpackungen miteinander verbunden sind und durch ein Abreißen voneinander getrennt werden können. Die Verpackungen sind innerhalb des Lagerungsbehälters aufgerollt. Mittels eines Bewegungssensors wird bewirkt, dass ein nachfolgendes verpacktes Sterilgut soweit aus der Ausgabeöffnung befördert wird, bis dieses durch ein Abreißen von einer nachfolgenden Verpackung abgetrennt werden kann.

Die US 20190224404 A1 offenbart eine Vorrichtung, die zur Lagerung, Ausgabe und Entsorgung von medizinischen Verbrauchsmaterialien wie Nahtmaterial/Nadeln dient. Die Vorrichtung kann das ausgegebene Verbrauchsmaterial zählen, das aus der Vorrichtung abgegeben wird. Die Vorrichtung kann so konfiguriert werden, dass sie vorverpacktes Nahtmaterial in der Originalverpackung oder speziell für die Vorrichtung entwickelte Kartuschen aufnehmen kann.

Weitere Vorrichtungen zur Lagerung und Ausgabe von medizinischem Verbrauchsmaterial oder Sterilgut sind bekannt aus der DE 102015112225 A1, der DE 29502945 U1**,** der EP 1876989 B1**,** der US 10864315 B2**,** der DE 202009009747 U1**,** der AU 2013389393 B2**,** der DE 9214287 U1**,** der US 20190076205 A1**,** der US 2020069405 A1**,** der US 2006243635 A1**,** der US 2020086063 A1 und der WO 2011097418 A2**.**

Die DE 102020216423 A1 offenbart eine Vorrichtung, die zur Desinfektion von Oberflächen und/oder Raumluft dient, wobei zumindest eine UV-Quelle in oder an einem medizinischen Gerät und/oder einem Ausrüstungsgegenstand eines medizinischen Untersuchungs- und/oder Behandlungsraums derart angeordnet ist, dass sie zur desinfizierenden Bestrahlung zumindest einer Oberfläche und/oder zumindest eines Luftstroms geeignet ist.

Die US 2019240089 A1 offenbart einen automatischen Spender zur Ausgabe von sterilen Hygieneartikeln für Frauen, wobei eine Vorwärtsbewegungseinheit dazu geeignet ist, den Hygieneartikel durch direktes Drücken mit der Vorwärtsbewegungseinheit aus einem Auslassschlitz nach vorne zu bewegen.

Die genannten Offenbarungen umfassen zwar Vorrichtungen, welche zur Lagerung von sterilen Gütern eingerichtet sind, allerdings liegen die sterilen Güter entweder jeweils einzeln verpackt innerhalb des Lagerungsbehältnisses vor oder sie liegen vereinzelt und unverpackt vor. Im ersten Fall fällt weiterhin eine erhebliche Menge an Verpackungsmüll an, da auf eine Sterilität wahrende Verpackungen nicht verzichtet wird. Im zweiten Fall ist eine Sterilität der gelagerten Güter auf Dauer nicht gewährleistet, was insbesondere der Tatsache geschuldet ist, dass die Behälter durch eine Öffnung mit nicht verpacktem Sterilgut befüllt werden oder durch die Entnahmeöffnung des Behälters die Wahrscheinlichkeit einer Kontamination des gelagerten Sterilguts erhöht ist.

Dem Stand der Technik mangelt es an einer Aufbewahrungs- und Ausgabevorrichtung für nicht verpackte, vereinzelte Sterilgüter, wobei die Sterilität der gelagerten Sterilgüter bevorzugt dauerhaft oder über einen längeren Zeitraum gewährleistet bleibt.

### AUFGABE

Die Aufgabe der vorliegenden Erfindung ist es, eine Vorrichtung für Sterilgut bereitzustellen, durch welche kein Verpackungsmüll für die sterile Lagerung von Sterilgut anfällt. Zudem soll die Erfindung eine berührungslose Entnahme des Sterilguts aus einem Sterilgut-Lagerungsbehälter ermöglichen, wobei die Gefahr einer möglichen Kontamination des gelagerten sowie des ausgegebenen Sterilguts, im Vergleich zu dem im Stand der Technik beschriebenen Vorrichtungen, erheblich reduziert ist.

### LÖSUNG

Die Aufgabe wird mit einer Sterilgut-Ausgabevorrichtung mit den Merkmalen des Hauptanspruches gelöst. Weitere vorteilhafte Ausgestaltungen der Erfindung sind den Unteransprüchen, der Beschreibung und den Ausführungsbeispielen zu entnehmen.

### ALLGEMEINE VORTEILE

Durch die Erfindung wird Verpackungsmüll vermieden, da auf Verpackungen gänzlich verzichtet wird, indem ein Sterilgut-Lagerungsbehälter mit einem zu sterilisierenden Gut, insbesondere einem Sterilgut, befüllt wird und dann mit dem darin gelagerten Sterilgut sterilisiert wird. Weiterhin wird durch die Erfindung eine kontaktlose Entnahme des Sterilguts aus dem Sterilgut-Lagerungsbehälter ermöglicht. D.h., die Oberfläche des Sterilgut-Lagerungsbehälters muss zur Entnahme des Sterilguts durch einen Nutzer nicht berührt werden. Die Möglichkeit, dass das Fassungsvolumen des Sterilgut-Lagerungsbehälters an die Menge des aufzubewahrenden Inhalts angepasst werden kann, stellt einen weiteren Vorteil dar.

### BESCHREIBUNG DER ERFINDUNG

Die Erfindung betrifft eine Sterilgut-Ausgabevorrichtung zum Lagern und Ausgeben von Sterilgut, aufweisend einen Sterilgut-Lagerungsbehälter sowie ein unteres Ausgabemodul, wobei der Sterilgut-Lagerungsbehälter für eine sterile Lagerung des Sterilguts eingerichtet ist, wobei das untere Ausgabemodul eine Ausgabeöffnung aufweist, die zur Ausgabe von Sterilgut eingerichtet ist, wobei der Sterilgut-Lagerungsbehälter dazu eingerichtet ist, mit dem Sterilgut befüllt zu werden, wobei der Sterilgut-Lagerungsbehälter dazu eingerichtet ist, mit oder ohne innerhalb des Sterilgut-Lagerungsbehälters gelagertem Sterilgut sterilisiert zu werden, wobei die Sterilgut-Ausgabevorrichtung zur berührungslosen Ausgabe von Sterilgut aus dem Sterilgut-Lagerungsbehälter eingerichtet ist, wobei der Sterilgut-Lagerungsbehälter und das untere Ausgabemodul dazu eingerichtet sind reversibel miteinander verbunden zu werden, wobei das Sterilgut erst nach seiner Ausgabe durch das untere Ausgabemodul einer nichtsterilen Umgebung exponiert ist, wobei das untere Ausgabemodul ein Sterilgut-Suspensionsmodul aufweist, welches dazu eingerichtet ist, das ausgegebene Sterilgut bis zu dessen Entnahme durch einen Nutzer mechanisch oder magnetisch zu suspendieren.

Bevorzugt dient die Sterilgut-Ausgabevorrichtung zum Lagern und Ausgeben von lose gelagertem, stückigen Sterilgut, wobei hierin die Begrifflichkeiten Sterilgut und stückiges Sterilgut äquivalent verwendet werden. Bei einem Sterilgut handelt es sich um ein Material, welches im medizinischen Routinebetrieb Verwendung findet. Bspw. handelt es sich bei einer Einmalspritze um ein Sterilgut.

Unter einem lose gelagerten, stückigen Sterilgut werden bevorzugt einzelne Gegenstände verstanden, welche voneinander getrennt sind und ein im Wesentlichen schüttfähiges Verhalten zeigen können. Ein Sterilgut kann verpackt sein, bevorzugt wird jedoch auf eine Verpackung verzichtet, um die Entstehung von Verpackungsmüll vorteilhaft zu vermeiden.

Bevorzugt liegt ein Sterilgut, das zur Ausgabe durch die Sterilgut-Ausgabevorrichtung vorgesehen ist, unverpackt vor und kann einem thermischen, chemischen und/oder physikalischen Sterilisationsverfahren unterworfen werden. Ein thermisches Sterilisationsverfahren umfasst Dampfsterilisation oder Heißluftsterilisation. Ein chemisches Sterilisationsverfahren umfasst Nassantiseptik (z.B. mit Wasserstoffperoxid, gelöstem Ozon, Peressigsäure) oder Trockenantispetik (z.B. mit Formaldehyd, Ethylenoxid, Ozon oder Wasserstoffperoxid). Ein physikalisches Sterilisationsverfahren umfasst Hochdrucksterilisation, Strahlensterilisation (z.B. mit ionisierender Strahlung umfassend UV-, Röntgenstrahlung, Gammastrahlung) oder Plasmasterilisation.

Die Sterilgut-Ausgabevorrichtung weist einen Sterilgut-Lagerungsbehälter auf, der dazu eingerichtet ist, das Sterilgut zu lagern. Bevorzugt ist eine sterile Lagerung des Sterilguts innerhalb des Sterilgut-Lagerungsbehälters möglich. Dies bedeutet, dass bei bestimmungsgemäßem Gebrauch bevorzugt keine oder nur eine sehr geringe Anzahl an Mikroorganismen oder Partikeln in das Innere des Sterilgut-Lagerungsbehälters vordringen kann. Besonders bevorzugt beugt der Sterilgut-Lagerungsbehälter einer Kontamination des darin aufbewahrten Sterilguts vor. Die Anzahl von eventuell in das Innere des sterilisierten Sterilgut-Lagerungsbehälters bei bestimmungsgemäßem Gebrauch vorgedrungenen Mikroorganismen liegt bevorzugt bei 1 oder weniger als 1 in einem Zeitraum von 1 Monat, bevorzugt 2 Monaten, ganz besonders bevorzugt 3 Monaten und ganz besonders bevorzugt mehr als 3 Monaten.

Der Sterilgut-Lagerungsbehälter kann abschnittsweise oder vollständig aus einem Metall (z.B. Edelstahl) oder einem Kunststoff gebildet sein. Bevorzugt ist der Sterilgut-Lagerungsbehälter aus einem Material gebildet, welches zumindest einem der oben genannten Sterilisationsverfahren mehrfach widersteht.

Ein Sterilgut-Lagerungsbehälter kann auch zumindest abschnittsweise aus einem organischen Polymer (z.B. Zellulose) gebildet sein, welches beschichtet, insbesondere mit einer weiter unten beschriebenen funktionalen Beschichtung beschichtet, sein kann.

Die Beschichtung kann so ausgestaltet sein, dass der Kunststoff oder das organische Polymer dem Sterilisationsverfahren widerstehen. Es wird bevorzugt ein solcher Kunststoff oder ein solches organisches Polymer verwendet, welches, gemäß bekannter Verfahren, recycelt werden kann. Somit kann das Material des Sterilgut-Lagerungsbehälters vorteilhaft wiederverwendet werden, wenn der Sterilgut-Lagerungsbehälter bspw. defekt ist oder ausgemustert wird. Es kann aber auch gewünscht sein, dass der Sterilgut-Lagerungsbehälter nicht wiederverwertet werden soll. Demnach ist dieser zumindest abschnittsweise oder vollständig bevorzugt aus einem biologisch abbaubaren Material, insbesondere einem biologisch abbaubaren Polymer, gebildet.

Eine Sterilisation zielt auf die Abtötung aller vorhandenen Mikroorganismen und Keime in einer Lösung, auf festen Oberflächen oder auch in der umgebenden Luft ab. Hierin sind unter Mikroorganismen Bakterien, Viren und Sporen, ferner infektiöse Proteine, wie Prionen, sowie Plasmide und andere pathogene DNA-Fragmente zu verstehen. Eine 100%ige Abtötung aller Mikroorganismen und Keime lässt sich in der Praxis allerdings nicht verwirklichen. Deswegen ist der Sterilisationserfolg, der durch ein bestimmtes Verfahren, insbesondere eines der obigen Sterilisationsverfahren, erzielt wird, stets nur als Wahrscheinlichkeit für die Verringerung der Zahl an lebenden Mikroorganismen und Keimen unterhalb eines Grenzwertes definierbar. Das Maß dafür ist die nach der Sterilisation noch nachweisbare Anzahl "koloniebildender Einheiten" (KBE, engl.: CFU = colony forming units), die übrig gebliebene Anzahl fertiler Mikroorganismen und Keime. Ein (Steril-)Gut, das nach einem bestimmten Sterilisationsverfahren behandelt wurde, wird praktisch als "steril" bezeichnet, wenn eine Reduktion der Keimzahl mindestens um den Faktor 10⁶ erreicht worden ist. D.h., dass von einer Million gleich behandelter, identischer Einheiten (eines Sterilguts) nur noch in einer Einheit koloniebildende Einheiten (KBE), d.h. vermehrungsfähige Keime, nachweisbar sein dürfen.

Die erfindungsgemäße Sterilgut-Ausgabevorrichtung weist ein unteres Ausgabemodul mit einer Ausgabeöffnung auf. Aus dem Stand der Technik sind Ausgabemodule bekannt, die bspw. am unteren Ende eines Sterilgut-Lagerungsbehälters angeordnet sind und bspw. eine einfache Öffnung, Klappe oder Schlitz umfassen, über diese das Sterilgut entnehmbar ist. Hierin ist unter einem Ausgabemodul der Bereich zu verstehen, der dem Sterilgut-Lagerungsbehälter nachfolgend angeordnet ist und der von dem Sterilgut passiert wird, um letztlich aus der erfindungsgemäßen Sterilgut-Ausgabevorrichtung entnommen werden zu können. Die Ausgabeöffnung kann dabei bevorzugt über eine weiter unten beschriebene Mechanik geöffnet und geschlossen werden. Innerhalb oder an dem Ausgabemodul ist bevorzugt ein weiter unten beschriebenes Sterilisationsmodul angeordnet, um den Bereich um und insbesondere die Ausgabeöffnung steril zu halten.

Die Befüllung des Sterilgut-Lagerungsbehälters mit einem Sterilgut kann über eine Öffnung (hierin auch: Sterilgut-Lagerungsbehälter-Öffnung) erfolgen, welche vom Sterilgut-Lagerungsbehälter umfasst ist. Bevorzugt lässt sich die Öffnung des Sterilgut-Lagerungsbehälters mit einem Verschlusselement abdichten. Weiter bevorzugt lässt sich die Öffnung so mit dem Verschlusselement abdichten, dass einer Passage von Mikroorganismen in den Sterilgut-Lagerungsbehälter vorteilhaft vorgebeugt wird. Das Verschlusselement kann im Wesentlichen aus einer Membran gebildet sein. Damit ist vorteilhaft ein Gasaustausch des vom Sterilgut-Lagerungsbehälter umfassten Volumens mit dem Volumen außerhalb des Sterilgut-Lagerungsbehälters ermöglicht. Nach der Befüllung des Sterilgut-Lagerungsbehälters kann dieser einem hierin genannten Sterilisationsverfahren unterzogen werden.

In einer bevorzugten Ausgestaltung ist der Sterilgut-Lagerungsbehälter mit dem Sterilgut nachfüllbar. Dabei ist es besonders bevorzugt, wenn der Sterilgut-Lagerungsbehälter über eine Sterilgut-Lagerungsbehälter-Öffnung befüllt werden kann, über welche das Sterilgut den Sterilgut-Lagerungsbehälter verlässt. Die Sterilgut-Lagerungsbehälter-Öffnung ist vorzugsweise auf der Seite des Sterilgut-Lagerungsbehälters angeordnet, welche dem Ausgabemodul zugewandt ist. Um den Sterilgut-Lagerungsbehälter zu befüllen, muss die zur Befüllung vorgesehene Sterilgut-Lagerungsbehälter-Öffnung für die Zeitdauer der Befüllung zugänglich sein, wofür der Sterilgut-Lagerungsbehälter von dem unteren Ausgabemodul separiert werden kann.

Die Lagerungsbehälter-Öffnung ist bei bestimmungsgemäßem Gebrauch bevorzugt an der Unterseite des Sterilgut-Lagerungsbehälters angeordnet. Sofern der Sterilgut-Lagerungsbehälter nicht, wie weiter unten beschrieben, mit dem Ausgabemodul verbindbar ist, werden die Begriffe "Sterilgut-Lagerungsbehälter-Öffnung" und "Ausgabeöffnung" äquivalent verwendet, da in diesem Fall das Ausgabemodul und der Sterilgut-Lagerungsbehälter Teil der gleichen baulichen Einheit sind und es nicht vorgesehen ist, dass beide voneinander separiert werden.

Gemäß einer Ausführungsform ist die Sterilgut-Lagerungsbehälter-Öffnung mit einem Abdeckmittel (z.B. abziehbare Folie) versehen. Bei einem Abdeckmittel kann es sich bspw. um eine gasdurchlässige Membran handeln. Diese kann vor dem Sterilisationsverfahren so auf die Öffnung aufgebracht werden, dass diese vorteilhaft so abgedichtet ist, dass keine Mikroorganismen in den Innenraum des Sterilgut-Lagerungsbehälters vordringen können, wobei eine Gaspassage, bspw. von Dampf während einer Dampfsterilisation, ermöglicht ist.

Das Abdeckmittel kann vor dem Verbinden des Sterilgut-Lagerungsbehälters mit dem unteren Ausgabemodul entfernt werden. Durch das Abdeckmittel wird vorteilhaft erreicht, dass die Gefahr einer Kontamination des im Sterilgut-Lagerungsbehälter gelagerten Inhalts zusätzlich vorgebeugt wird.

Die hierin verwendeten Begriffe "oben" und "unten" beziehen sich auf die allgemein anerkannten räumlichen Punkte in Bezug auf einen Gegenstand in weiterem Bezug zum Schwerefeld der Erde.

Es sind zudem weitere Öffnungen zur Befüllung des Sterilgut-Lagerungsbehälters mit Sterilgut denkbar. Dabei kann eine weitere Öffnung auch oben oder an einer Seite des Sterilgut-Lagerungsbehälters angeordnet sein. Jedoch werden weitere Öffnungen dann weniger bevorzugt, wenn diese eine erhöhte Gefahr von Kontaminationen des Behälterinneren des Sterilgut-Lagerungsbehälters bergen. Allerdings kann eine weitere Öffnung auch bevorzugt sein, wenn diese bspw. dem Einleiten oder Ausleiten eines Sterilisationsmittels (Gas oder Flüssigkeit) in oder aus den Sterilgut-Lagerungsbehälter dient. So sind aus dem Stand der Technik Behältnisse bekannt, die zumindest eine Öffnung umfassen, welche mit einem feinporigen Sieb abgedeckt sind, wobei das Sieb bspw. Gase oder Flüssigkeiten, aber keine Mikroorganismen passieren lässt.

Die erfindungsgemäße Sterilgut-Ausgabevorrichtung zeichnet sich unter anderem dadurch aus, dass der Sterilgut-Lagerungsbehälter mit einem der oben genannten Sterilisationsverfahren sterilisierbar ist. Zudem ist vorgesehen, dass der Sterilgut-Lagerungsbehälter, insbesondere mit darin gelagertem Sterilgut, zumindest einmalig, bevorzugt mehrfach, sterilisierbar ist.

Weiterhin zeichnet sich die erfindungsgemäße Sterilgut-Ausgabevorrichtung dadurch aus, dass das Sterilgut aus dem Sterilgut-Lagerungsbehälter berührungslos ausgegeben werden kann. Darunter ist zu verstehen, dass ein Nutzer einzelne Teile der erfindungsgemäßen Vorrichtung, insbesondere den Sterilgut-Lagerungsbehälter und das untere Ausgabemodul, nicht berühren muss, um zumindest ein Sterilgut aus dem Sterilgut-Lagerungsbehälter zu erhalten. Es kann aber auch vorgesehen sein, dass einzelne Teile der Sterilgut-Ausgabevorrichtung (z.B. die Außenwandung des Sterilgut-Lagerungsbehälters) berührt werden können.

Um eine berührungslose Ausgabe von Sterilgut aus dem Sterilgut-Lagerungsbehälter zu ermöglichen, kann die Sterilgut-Ausgabevorrichtung einen Sensor, insbesondere einen Annäherungssensor, umfassen.

Gemäß einer bevorzugten Ausführungsform weist das untere Ausgabemodul eine Elektronik mit zumindest einem Annäherungssensor auf. Eine Elektronik beschreibt dabei eine frei gestaltbare Anordnung von elektronischen Baugruppen, welche den Annährungssensor einerseits mit elektrischem Strom/Spannung versorgt und andererseits dazu ausgelegt ist, dass durch den Annäherungssensor bewirkte Signal zu verarbeiten. Der Annäherungssensor und die damit verschaltete Elektronik sind bevorzugt dazu ausgelegt, die Annäherung einer Extremität, wie bspw. einer Hand, des Nutzers zu detektieren. Dem Fachmann sind Annäherungssensoren hinreichend bekannt. Diese nutzen für gewöhnlich Teile des elektromagnetischen Spektrums (z.B. Infrarotlicht, Laser, elektrische Felder) oder Schall.

Bevorzugt wird erst durch die Annäherung der Extremität, wie bspw. einer Hand, eines Nutzers das Sterilgut ausgegeben. Die berührungslose Ausgabe des Sterilguts erfordert zumindest einen Annäherungssensor und eine Mechanik, die vom Fachmann frei gestaltet werden kann. Im einfachsten Fall ist die Ausgabeöffnung/Öffnung mit einer durch einen Aktor bewegten Klappe verschlossen, welche sich öffnet, sobald die Elektronik, über den Annäherungssensor, die Hand eines Nutzers registriert hat. Die elektronische Zusammenschaltung von Sensor, Aktor und weiteren mechanischen Elementen kann vom Fachmann frei ausgewählt werden.

In einer weiteren Ausgestaltung ist vorgesehen, dass, statt der Hand eines Nutzers, bspw. die bloße Anwesenheit des Nutzers zur Ausgabe des Sterilguts aus dem Sterilgut-Lagerungsbehälter führt. Der Annäherungssensor ist hierfür so ausgestaltet, dass er die Präsenz des Nutzers, insbesondere die Annäherung eines Transponders, oder einer gleichwertigen Vorrichtung (z.B. einem Smartphone) mit aktiver und/oder passiver Sendefunktion erfasst und dies an die Elektronik übermittelt, um einen Auswurf / eine Ausgabe des Sterilguts zu bewirken. Durch die Verwendung eines Transponders oder einer anderen Identifikationseinheit kann vorteilhaft erreicht werden, dass nur Nutzer, die zur Ausgabe von Sterilgut autorisiert sind, Sterilgut entnehmen können.

Gemäß einer bevorzugten Ausführungsform sind der Sterilgut-Lagerungsbehälter und das untere Ausgabemodul dazu eingerichtet reversibel miteinander verbunden zu werden. Unter einer solchen reversiblen Verbindung wird verstanden, dass der Sterilgut-Lagerungsbehälter und das untere Ausgabemodul so ausgestaltet sind, dass sie bevorzugt zumindest partiell formschlüssig verbunden werden können.

Um die reversible Verbindung zu ermöglichen, umfasst das untere Ausgabemodul und der Sterilgut-Lagerungsbehälter ein entsprechend ausgestaltetes Verbindungsmittel. Bei einem solchen Verbindungsmittel kann es sich um ein magnetisches Verbindungsmittel, ein Klicksystem, ein Stecksystem, ein Schienensystem, und/oder einer Kombination aus zumindest einem der genannten handeln, wobei das Verbindungsmittel eine reversible Verbindung des Sterilgut-Lagerungsbehälters mit dem unteren Ausgabemodul ermöglicht. Dem Fachmann sind weitere Verbindungsmittel bekannt.

Unter einem Klicksystem wird hierin ein Stecksystem verstanden, welches über einen Schnappmechanismus verfügt, der eine rückwärtige Bewegung der ineinandergesteckten Elemente (z.B. Sterilgut-Lagerungsbehälter mit unterem Ausgabemodul) nicht gestattet, es sein denn, der Schnappmechanismus wird aktiv vom Nutzer gelöst, beispielsweise an zumindest an einer Stelle gedrückt, um den entstandenen Formschluss wieder zu lösen. Verschiedene Klicksysteme sind dem Fachmann hinreichend bekannt.

In einer Ausführungsform ist das untere Ausgabemodul direkt an einer Wand oder an dem Ausgabemodul-Halterungsmittel befestigt. Der Sterilgut-Lagerungsbehälter kann dann mit dem Ausgabemodul verbunden werden, bevorzugt auf das untere Ausgabemodul geschoben werden. Während dieses Vorgangs kann eine vom Sterilgut-Lagerungsbehälter umfasste Mechanik dazu eingerichtet sein, einen Verschlussmechanismus zu öffnen, welcher dafür sorgt, dass das Innere des Sterilgut-Lagerungsbehälters (das Fassungsvolumen) mit dem Inneren des unteren Ausgabemoduls fluidverbunden wird und das auszugebende Sterilgut aus dem Sterilgut-Lagerungsbehälter in das untere Ausgabemodul gelangen kann. Die genaue Ausgestaltung dieser Mechanik wird der Fachmann so wählen, dass die Sterilität zumindest des Inneren des Sterilgut-Aufnahmebehälters nicht gefährdet ist und das darin gelagerte Sterilgut nicht kontaminiert wird.

Gemäß einer Ausgestaltung ist das untere Ausgabemodul mit einem Ausgabemodul-Halterungsmittel reversibel verbindbar. Unter einem Ausgabemodul-Halterungsmittel wird hierin eine Vorrichtung verstanden, welche form-, kraft- oder stoffschlüssig beispielsweise an einer Wand befestigt ist (z.B. über Stecken, Schauben, Kleben) und dazu eingerichtet ist, dass das untere Ausgabemodul am Ausgabemodul-Halterungsmittel befestigt werden kann. Besonders bevorzugt ist das untere Ausgabemodul reversibel mit dem Ausgabemodul-Halterungsmittel verbindbar. Dies kann beispielsweise über eine Steckverbindung erfolgen. So kann das Ausgabemodul-Halterungsmittel und das untere Ausgabemodul einen Klick- und/oder Schienenmechanismus umfassen, um die reversible Verbindung zu ermöglichen.

Zusätzlich kann vorgesehen sein, dass auch der Sterilgut-Lagerungsbehälter mit dem Ausgabemodul-Halterungsmittel verbindbar, bevorzugt reversibel verbindbar ist. Hierfür muss das Ausgabemodul-Halterungsmittel so ausgestaltet sein, dass es die Dimensionen des Sterilgut-Lagerungsbehälters und des unteren Ausgabemoduls ausreichend abdeckt.

In einer alternativen Ausführungsform kann der Sterilgut-Lagerungsbehälter auch dadurch an einer Wand oder einem anderen Halterungsort befestigt werden, dass der Sterilgut-Lagerungsbehälter ein Haltemittel aufweist, welches vorzugsweise als mit einem an einer Wand befestigten Haltemittel eingreifend ausgestaltet ist.

Gemäß einer bevorzugten Ausgestaltung ist das untere Ausgabemodul, oder das untere Ausgabemodul und der Sterilgut-Lagerungsbehälter, in einer Raumhöhe positionierbar. Unter einer Raumhöhe wird jeder Punkt in einem Raum verstanden, der nicht mit dem Boden oder der Raumdecke deckungsgleich ist. Bevorzugt ist das Ausgabemodul so in einer Raumhöhe positioniert (auch: angeordnet) oder positionierbar (auch: anordenbar), so dass es einem durchschnittlich großen Nutzer ermöglicht ist, die Sterilgut-Ausgabevorrichtung komfortabel zu bedienen. In einer bevorzugten Ausgestaltung ist die Position des unteren Ausgabemoduls in zumindest zwei Raumhöhen einstellbar. Somit kann die Lage des Ausgabemoduls vorteilhaft an die Größe unterschiedlich großer Nutzer angepasst werden.

Das Sterilgut ist bevorzugt erst nach seiner Ausgabe durch das untere Ausgabemodul einer nichtsterilen Umgebung exponiert. Dabei ist unter einer nichtsterilen Umgebung jeder Raumbereich zu verstehen, der nicht identisch zum Innenraum des Sterilgut-Lagerungsbehälters ist, in welchem das Sterilgut lagert.

Gemäß einer bevorzugten Ausführungsform ist das Volumen, insbesondere das Fassungsvolumen des Sterilgut-Lagerungsbehälters veränderbar. Dies bedeutet, dass das Volumen, insbesondere das Fassungsvolumen, vergrößert und/oder verkleinert werden kann. Somit kann das Volumen des Sterilgut-Lagerungsbehälters vorteilhaft an dessen Inhalt, also die zur Lagerung vorgesehene, aufzunehmende oder aufgenommene Menge an Sterilgut angepasst werden. Ein kleineres Volumen hat zur Folge, dass weniger Volumen sterilisiert werden muss, was vorteilhaft die Effizienz des Sterilisationsverfahrens erhöht. Unter dem Fassungsvolumen wird das Volumen verstanden, welches vom Sterilgut-Lagerungsbehälter umfasst ist und in welchem das Sterilgut gelagert ist.

Um das Fassungsvolumen (hierin auch: Volumen) des Sterilgut-Lagerungsbehälters veränderlich zu gestalten, ist der Sterilgut-Lagerungsbehälter aus zumindest zwei Behälterhälften gebildet, wobei die Behälterhälften dazu eingerichtet sind, gegeneinander verschoben zu werden. Durch diese Verschiebung kann das Fassungsvolumen des Sterilgut-Lagerungsbehälters vorteilhaft an die Menge des von diesem aufzunehmenden Sterilguts angepasst werden. Damit kann bspw. erreicht werden, dass ein Sterilgut-Lagerungsbehälter abhängig von seinem Füllstand andere Abmessungen erreicht.

Um das Volumen veränderbar zu gestalten, kann der Sterilgut-Lagerungsbehälter im Wesentlichen aus zwei Behälterhälften gebildet sein, wobei diese so ausgestaltet sind, dass sie ein teleskopartiges Verschieben einer oberen Behälterhälfte gegen eine untere Behälterhälfte ermöglichen, um das Fassungsvolumen des Sterliguts-Lagerungsbehälters zu vergrößern oder zu verkleinern.

Gemäß einer weiteren Ausführungsform weist die erfindungsgemäße Vorrichtung einen Sensor auf, der dazu eingerichtet ist, das Volumen des Sterilgut-Lagerungsbehälters zu erfassen und dieses an eine Recheneinheit zu übermitteln.

Gemäß einer Ausführungsform weist die Sterilgut-Ausgabevorrichtung ein Ausgabemodul-Halterungsmittel auf, welches für eine reversible Verbindung mit dem Ausgabemodul eingerichtet ist, wobei das Ausgabemodul-Halterungsmittel einen ersten elektrischen Kontaktpunkt und das untere Ausgabemodul einen zweiten elektrischen Kontaktpunkt aufweist.

Das Ausgabemodul-Halterungsmittel ist dazu eingerichtet das untere Ausgabemodul in einer Raumhöhe zu positionieren, wobei das Ausgabe-Halterungsmittel selbst in einer Raumhöhe positioniert oder durch einen Nutzer positionierbar ist. Bspw. kann das Ausgabe-Halterungsmittel im Wesentlichen aus zumindest einer senkrechten Schiene gebildet sein, wobei das untere Ausgabemodul auf dieser Schiene positioniert werden kann.

Die reversible Verbindung des Ausgabemodul-Halterungsmittels mit dem unteren Ausgabemodul kann über ein magnetisches Verbindungsmittel, oder ein Stecksystem, oder ein Schienensystem oder eine Kombination aus zumindest einem der genannten realisierbar werden.

Gemäß einer bevorzugten Ausgestaltung weist das Ausgabemodul-Halterungsmittel und/oder das untere Ausgabemodul zumindest einen elektrischen Kontaktpunkt (hierin auch: elektrischer Kontakt) auf. Dabei sind diese so angeordnet, dass sich die elektrischen Kontakte kontaktieren, sobald das untere Ausgabemodul mit dem Ausgabemodul-Halterungsmittel verbunden ist (auch: reversible elektrische Verbindung). Die elektrischen Kontakte, die von dem, bspw. an einer Wand befestigten, Ausgabemodul-Halterungsmittel umfasst sind, sind bevorzugt mit einer Stromversorgung verbunden, die in oder entlang einer Wand oder der oben beschriebenen Schiene (auch: Halterungsschiene) geführt ist, wobei die Schiene bevorzugt an der Wand angeordnet ist. Durch das Verbinden des unteren Ausgabemoduls mit dem Ausgabemodul-Halterungsmittel und dem folgenden Kontaktieren der elektrischen Kontakte, kann eine Stromversorgung in das untere Ausgabemodul geführt und dort bereitgestellt werden.

In einer weiteren Ausführungsform kann das Ausgabemodul-Halterungsmittel und/oder das untere Ausgabemodul eine bevorzugt auswechselbare Energiespeichereinrichtung wie eine Batterie oder ein Akkumulator aufweisen, um eine Energieversorgung des unteren Ausgabemoduls und/oder des Sterilgut-Lagerungsbehälters vorteilhaft vom Stromnetz unabhängig zu machen. Dabei kann der Akkumulator über die elektrischen Kontakte geladen werden. Das Vorhandensein einer Batterie, oder eines Akkumulators, oder einer gleichwertigen Einrichtung ermöglicht vorteilhaft, dass die Ausgabe von Sterilgut auch dann ermöglicht ist, wenn das Energieversorgungsnetz ausgefallen ist.

Beispielsweise kann mit der Energieversorgung / der bereitgestellten elektrischen Energie eine von der erfindungsgemäßen Vorrichtung umfasste Elektronik, ein Annäherungssensor, ein Aktuator, eine Elektromechanik, und/oder ein Leuchtmittel (z.B. LEDs, Displays) mit elektrischer Energie versorgt werden, um das Sterilgut auszuwerfen. Um die Sicherheit zu erhöhen, kann die Stromversorgung im Niedervoltbereich (z.B. 12V) bereitgestellt werden.

In einer Ausführungsform kann auch zwischen dem unteren Ausgabemodul und dem Sterilgut-Lagerungsbehälter eine elektrische Verbindung, bevorzugt eine reversible elektrische Verbindung, hergestellt werden, um bspw. die weiter unten beschriebenen Elemente mit elektrischer Energie zu versorgen.

Gemäß einer bevorzugten Ausführungsform ist eine erfolgte reversible Verbindung dazu eingerichtet, den ersten elektrischen Kontaktpunkt mit dem zweiten elektrischen Kontaktpunkt zu kontaktieren. Bevorzugt werden erst durch die reversible Verbindung, bspw. durch ein Aufstecken oder Aufschieben des Sterilgut-Lagerungsbehälters auf das untere Ausgabemodul, die elektrischen Kontaktpunkte/Kontakte freigegeben. Somit ist die Sicherheit in der Handhabung der erfindungsgemäßen Vorrichtung erhöht, da einem Stromschlag eines Nutzers so wirksam vorgebeugt wird.

Gemäß einer Ausführungsform weist das untere Ausgabemodul ein Sterilgut-Suspensionsmodul auf, welches dazu eingerichtet ist, das ausgegebene Sterilgut bis zu dessen Entnahme durch einen Nutzer mechanisch oder magnetisch zu suspendieren.

In einer weiteren Ausführungsform weist das untere Ausgabemodul ein Sterilgut-Suspensionsmodul auf. Dieses ist dazu eingerichtet, das durch die Ausgabeöffnung des Ausgabemoduls ausgegebene Sterilgut zu suspendieren (hierin auch als halten oder aufhängen zu verstehen) bis es von einem Nutzer aus dem Sterilgut-Suspensionsmodul (hierin auch: Suspensionsmodul) entnommen wird. Ein Suspensionsmodul ist also im Prinzip eine Vorrichtung zum Halten des Sterilgutes, wobei dieses Halten vorzugsweise entweder mechanisch, beispielsweise durch eine nachfolgend beschriebene Halterung, oder magnetisch, realisierbar ist.

In einer weiteren Ausführungsform ist das Sterilgut-Suspensionsmodul derart ausgestaltet, dass es zumindest ein magnetisches Halterungselement aufweist. Das magnetische Halterungselement ist dazu eingerichtet, das ausgegebene Sterilgut bis zu dessen Entnahme durch einen Nutzer mittels magnetischer Wechselwirkungen zu suspendieren / zu halten, wobei eine magnetische Wechselwirkung hierin auch als eine kraftschlüssige Verbindung angesehen wird.

Bevorzugt ist das magnetische Halterungselement selbst magnetisch. Dabei kann es sich um einen Magneten wie einen Elektromagneten oder um einen Permanentmagneten (z.B. Neodym-Magnet) handeln. Sofern es sich um einen Elektromagneten handelt, kann die Stromversorgung zum Betrieb des Elektromagneten über weiter die hierin beschriebenen elektrischen Kontakte bereitgestellt werden.

Damit das aus dem Sterilgut-Lagerungsbehälter ausgegebene Sterilgut von einem Magneten gehalten werden kann, muss es entweder selbst einen Permanentmagneten aufweisen, oder bevorzugt ein Suspensionsmittel aufweisen, welches mit einem magnetischen Halterungselement, welches einen Magneten aufweist, eine magnetische Wechselwirkung eingeht.

In einer bevorzugten Ausführungsform weist das Sterilgut als Suspensionsmittel daher ein Metall (z.B. Stahl) auf, wobei das Metall bspw. als Metallring das Sterilgut umgeben kann oder im Material des Sterilgutes eingelassen ist. So kann es sich bei dem Sterilgut bspw. um eine Einmalspritze handeln, um deren im Wesentlichen zylinderförmigen Körper ein Metallring gelegt ist. Bevorzugt ist der Metallring so am Sterilgut angeordnet, dass die Bedienung des Sterilguts (bspw. das Ablesen einer Skala auf einer Einmalspritze) nicht beeinträchtigt ist. Dabei ist das Metallstück bevorzugt so angeordnet, dass es ohne großen Kraftaufwand von dem Sterilgut abgetrennt werden kann. Vorteilhaft kann damit erreicht werden, dass das Metallstück zum Anbringen an weiteres Sterilgut wiederverwertet werden kann.

Selbstverständlich kann das Sterilgut zumindest abschnittsweise oder gänzlich aus diesem Metall gebildet sein. Weiterhin bevorzugt handelt es sich bei dem Metall um ein Metall, welches mittels zumindest einem der oben genannten Sterilisationsverfahren bevorzugt mehrfach sterilisierbar ist.

Durch die magnetische Suspension des Sterilgutes nach der Ausgabe aus dem Sterilgut-Lagerungsbehälter kann die Kontaktfläche des ausgegebenen Sterilguts mit einer nicht sterilen Oberfläche vorteilhaft auf null reduziert werden, solange das ausgegebene Sterilgut durch das magnetische Halterungselement in der Schwebe gehalten wird. Damit ist die Gefahr einer Kontamination eines ausgegebenen Sterilgutes vorteilhaft erheblich minimiert.

Mit der Verwendung eines magnetischen Halterungselements kann zudem vorteilhaft erreicht werden, dass das Ausgeben des Sterilgutes geräuscharm erfolgt. Angenommen bei dem Sterilgut-Suspensionsmodul würde es sich lediglich um eine Schale handeln, in welche das Sterilgut nach der Ausgabe hineinfällt, so würde beim Auftreffen des Sterilgutes auf die Schale ein Geräusch erzeugt werden. Um die Entstehung eines Geräusches zu verhindern, kann das Sterilgut-Suspensionsmodul so ausgestaltet sein, dass zumindest ein magnetisches Halterungselement von einem im Wesentlichen vertikalen Fallmodul umfasst ist. Während des Falls des Sterilguts, wird dieses entlang des vertikalen Fallmodules abgebremst, bis das Sterilgut durch die magnetischen Wechselwirkungen in einer Höhe suspendiert ist.

Gemäß einer bevorzugten Ausgestaltung kann die magnetische Suspension des Sterilgutes über eine von der Sterilgut-Ausgabevorrichtung umfassten Elektronik gesteuert werden, wie es etwa von freischwebenden Globen bekannt ist, die als Dekorationsgegenstände Verwendung finden.

Das Sterilgut-Suspensionsmodul kann bspw. als eine Halterung ausgestaltet sein, in welche das Sterilgut fällt, sobald es die Ausgabeöffnung oder die Lagerungsbehälter-Öffnung verlassen hat. Die Halterung ist bevorzugt so ausgestaltet, dass das Sterilgut gegen ein ungewolltes Herausfallen gesichert ist. Im einfachsten Fall handelt es sich bei dem Sterilgut-Suspensionsmodul um eine Schale, in welche das Sterilgut fallen kann.

In einer weiteren Ausführungsform weist das untere Ausgabemodul zumindest zwei mechanische Kontaktpunkte auf, wobei diese für eine mechanische Suspension von ausgegebenem Sterilgut eingerichtet sind. Die mechanischen Kontaktpunkte sind einerseits dazu eingerichtet den vertikalen Fall des aus dem Sterilgut-Lagerungsbehälter ausgegebenen Sterilguts zu begrenzen und andererseits die laterale Bewegung des aus dem Sterilgut-Lagerungsbehälter ausgegebenen Sterilgutes zu begrenzen.

Damit dienen die mechanischen Kontaktpunkte der mechanischen Suspension des Sterilgutes.

Gemäß einer bevorzugten Ausgestaltung weist das Sterilgut-Suspensionsmodul zumindest eine Suspensionsschiene (hierin auch: Halterungsschiene) auf, wobei diese für eine mechanische Suspension von ausgegebenem Sterilgut eingerichtet ist. Eine mechanische Suspension eines Sterilgutes ist auch ohne magnetische Wechselwirkungen zwischen Sterilgut und Sterilgut-Suspensionsmodul möglich. Es kann aber auch vorgesehen sein, dass eine magnetische und mechanische Suspension miteinander kombiniert werden.

Die Suspensionsschiene ist bspw. aus zwei Halterungsschienen gebildet, welche derart ausgestaltet sind, dass eine vertikale Bewegung des ausgegebenen Sterilgutes innerhalb des Sterilgut-Suspensionsmoduls ermöglicht ist. Vorzugsweise wird eine laterale Bewegung des Sterilgutes innerhalb des Sterilgut-Suspensionsmoduls formschlüssig begrenzt. Damit wird vorteilhaft ein laterales Herausfallen von mechanisch suspendiertem Sterilgut aus dem Sterilgut-Suspensionsmodul verhindert.

Damit das ausgegebene Sterilgut nicht einfach nach Ausgabe aus dem Sterilgut-Lagerungsbehälter durch das Sterilgut-Suspensionsmodul hindurchrutscht, weist die untere Begrenzung des Sterilgut-Suspensionsmoduls, insbesondere die Halterungsschienen, eine Stoppvorrichtung auf, welche den vertikalen Fall des Sterilgutes begrenzt.

Ein Nutzer kann das Sterilgut dann auf einfache Weise aus dem Sterilgut-Suspensionsmodul entnehmen, indem er bei Entnahme des Sterilgutes dieses in einem Winkel anordnet, um das Sterilgut aus dem Sterilgut-Suspensionsmodul, insbesondere aus den Halterungsschienen zu entnehmen.

In einer weiteren Ausführungsform fällt das ausgegebene Sterilgut direkt in eine Hand des Anwenders/Nutzers. Vorzugsweise ist das Sterilgut-Suspensionsmodul mit einer weiter unten beschriebenen photokatalytischen Beschichtung beschichtet werden, so dass einer Kontamination des Sterilgut-Suspensionsmoduls durch ein versehentliches Berühren durch den Anwender vorgebeugt werden kann.

Gemäß einer bevorzugten Ausführungsform weist das Sterilgut-Suspensionsmodul ein magnetisches Suspensionselement (hierin auch: magnetisches Halterungselement) auf, welches dazu eingerichtet ist, das ausgegebene Sterilgut bis zu dessen Entnahme durch einen Nutzer magnetisch zu suspendieren oder in einer magnetischen Schwebe zu halten. Die Wirkungsweise dieser Merkmale ist weiter oben beschrieben.

Gemäß einer Ausführungsform ist das untere Ausgabemodul schwenkbar gegenüber dem Sterilgut-Lagerungsbehälter gelagert ist, sodass ein Winkel zwischen einer Mittelachse der Ausgabeöffnung und einer Längsachse des Sterilgut-Lagerungsbehälters einstellbar ist.

Die Längsachse des Sterilgut-Lagerungsbehälters ist als die Achse definiert, welche bei bestimmungsgemäßem Gebrauch, also dem Ausgeben des Sterilgutes aus dem Sterilgut-Lagerungsbehälter, im Wesentlichen zum Erdmittelpunkt orientiert ist. Eine Mittelachse der Ausgabeöffnung ist als die Achse zu verstehen, welche orthogonal zu einer Fläche und im Zentrum dieser Fläche steht, welche durch den Umriss der Ausgabeöffnung aufgespannt wird. Die Mittelachse kann im Wesentlichen parallel zur Längsachse orientiert sein. Der Umriss der Ausgabeöffnung bevorzugt als rechteckige Form ausgebildet. In einer Ausführungsform ist ein Winkel zwischen der Mittelachse der Ausgabeöffnung und der Längsachse des Sterilgut-Lagerungsbehälters einstellbar. Bevorzugte Winkelbereiche liegen im Bereich von 0° bis 90°, bevorzugt 0° bis 135° und ganz besonders bevorzugt von 0° bis 180°. Die Einstellbarkeit kann dadurch erreicht werden, indem das untere Ausgabemodul, insbesondere die Ausgabeöffnung, schwenkbar in Bezug auf die Längsachse des Sterilgut-Lagerungsbehälters ausgestaltet ist.

Dadurch kann vorteilhaft erreicht werden, dass das auszugebende Sterilgut nicht einfach entlang der Schwerkraft aus dem Sterilgut-Lagerungsbehälter fällt, sondern stattdessen umgelenkt wird. Dies bietet sich beispielsweise bei schwerem Sterilgut (z.B. mit Flüssigkeiten befüllte Behältnisse) an, so dass diese vorteilhaft nicht direkt auf ein Sterilgut-Suspensionsmodul prallen, sondern ein Teil der kinetischen Energie von der Wandung des Ausgabemoduls aufgenommen wird. Dabei kann die Wandung des Ausgabemoduls federnde Elemente umfassen, um vorteilhaft die Dämpfungseigenschaften des Ausgabemoduls zu erhöhen.

Weiterhin kann durch den einstellbaren Winkel erreicht werden, dass die Ausgabeöffnung besser einsehbar ist und der Nutzer die Bewegung und den Zeitpunkt des Erhalts des aus dem Sterilgut-Lagerungsbehälter ausgegebenen Sterilgutes vorteilhaft besser einschätzen kann. Aus dem gleichen Grund ist in einer weiteren Ausführungsform das Ausgabemodul zumindest teilweise transparent.

Gemäß einer bevorzugten Ausführungsform weist das untere Ausgabemodul eine Elektronik mit einem Annäherungssensor auf, wobei die Elektronik dazu eingerichtet ist, bei Annäherung einer Hand eines Anwenders an den Annäherungssensor eine Ausgabe des Sterilguts aus dem Sterilgut-Lagerungsbehälter zu bewirken.

Gemäß einer Ausgestaltung weist die Sterilgut-Ausgabevorrichtung, insbesondere das untere Ausgabemodul, und/oder eine Wandvorrichtung ein Sterilisationsmodul auf, wobei das Sterilisationsmodul zu einer Emission einer elektromagnetischen Welle und dazu eingerichtet ist das vom Sterilgut-Suspensionsmodul mechanisch oder magnetisch suspendierte Sterilgut mit einer elektromagnetischen Welle zu bestrahlen.

Das untere Ausgabemodul und/oder eine Wandvorrichtung und/oder der Sterilgut-Lagerungsbehälter kann ein Sterilisationsmodul aufweisen. Dabei ist insbesondere die oben beschriebene Ausgestaltung um die elektrischen Kontaktpunkte vorteilhaft, da das untere Ausgabemodul somit selbst keine Energiequelle (Batterie, Akku) umfassen muss.

Unter einer Wandvorrichtung wird hierin eine Vorrichtung verstanden, welche (ähnlich oder gleich wie das Ausgabemodul-Halterungsmittel) in einer Raumhöhe, bevorzugt an einer Wand oder einem anderen Mittel (z.B. Stativ) befestigt ist oder befestigt werden kann. Bei der Wandvorrichtung handelt es sich bevorzugt um eine Vorrichtung, welche die reversible Befestigung eines Sterilisationsmoduls erlaubt oder ein Sterilisationsmodul umfasst. Ein Sterilisationsmodul beschreibt eine Vorrichtung, welche zu einer Reduzierung der Anzahl an Mikroorganismen oder zumindest ein Hemmen des Wachstums von Mikroorganismen in der näheren Umgebung des Sterilisationsmoduls, zumindest jedoch im Bereich des unteren Ausgabemoduls, insbesondere der Ausgabeöffnung, eingerichtet ist.

Das Sterilisationsmodul ist bevorzugt zu einer Emission einer elektromagnetischen Welle eingerichtet. Eine erfindungsgemäße elektromagnetische Welle ist dazu eingerichtet, Mikroorganismen (z.B. Bakterien, Viren, Pilze) negativ zu beeinträchtigen, also zumindest im Wachstum zu hindern, bevorzugt diese aber abzutöten. Weiterhin bevorzugt ist eine elektromagnetische Welle dafür eingerichtet Proteine zu degradieren. Dabei ist dem Fachmann klar, dass die Fähigkeit einer elektromagnetischen Welle einen Mikroorganismus negativ zu beeinträchtigen, stark mit der Intensität, der Energie, also Wellenlänge, und der Dauer der emittieren elektromagnetischen Welle korreliert.

Die elektromagnetische Welle ist bevorzugt ausgewählt aus dem elektromagnetischen Spektrum umfassend Röntgenstrahlung, oder Mikrowellenstrahlung, oder ultraviolettem Licht, oder Blaulicht, oder einer Kombination aus zumindest einem der genannten. Durch eine Kombination zumindest einer der genannten ergänzen sich die antimikrobiellen Eigenschaften der jeweiligen Strahlungsspektren synergistisch.

Dem Fachmann sind Bereiche des elektromagnetischen Spektrums bekannt, welche eine schädigende Wirkung auf Mikroorganismen und/oder Proteine haben. Von Blaulicht ist bspw. bekannt, dass es im Wellenlängenbereich von 400 bis 450 Nanometer (nm) Mikroorganismen wirkungsvoll abtöten kann. Dies funktioniert über die Absorption des Blaulichts durch Biomoleküle (sogenannte Porphyrine), die in lebenden Zellen allgegenwärtig sind. Bei Anwesenheit von Sauerstoff bilden diese durch das Blaulicht energetisch angeregten Biomoleküle in einer chemischen Reaktion Sauerstoffradikale, die aufgrund ihrer hohen Reaktivität zu Zellschäden, der Verhinderung der Vermehrung oder zur Abtötung der Mikroorganismen führen.

Für die erfindungsgemäße Vorrichtung werden die folgenden Bereiche des elektromagnetischen Spektrums als vorteilhaft für die bestimmungsgemäße Verwendung (negatives Beeinträchtigen von Mikroorganismen/ Degradation von Proteinen) angesehen: Röntgenstrahlung mit einer Wellenlänge von 30 pm bis 1 nm, Mikrowellenstrahlung mit einer Wellenlänge von 1 mm bis 1 m, ultraviolettes Licht (z.B. UV-A, UV-B, besonders bevorzugt UV-C) mit einer Wellenlänge von 1 nm bis 380 nm, und/oder Blaulicht im Wellenlängenbereich von 380 bis 500 nm. Es ist dem Fachmann klar, dass die Emission zumindest der drei erstgenannten Strahlungsarten so erfolgen muss, dass ein Nutzer oder eine andere Person nicht von der Strahlung kontaktiert wird, um einen zellschädigenden Einfluss der Strahlung auf das Gewebe des Nutzers oder einer anderen Person zu verhindern.

Dies kann bspw. durch eine entsprechende bauliche Gestaltung der Sterilgut-Ausgabevorrichtung, insbesondere des unteren Ausgabemoduls realisiert werden. Vorzugsweise ist eine strahlungsabschirmende Schutzvorrichtung an der Sterilgut-Ausgabevorrichtung angeordnet. Weiterhin kann bspw. bei Annäherung der Hand eines Nutzers die emittierte elektromagnetische Welle bis zum Entfernen der Hand abgeschaltet oder in der Intensität reduziert werden. Vorteilhaft werden somit mögliche Zellschädigungen des Nutzers verhindert.

Weiterhin ist auch eine Kombination aus zumindest einer der oben genannten elektromagnetischen Wellen denkbar, um vorteilhaft ein breiteres elektromagnetisches Spektrum zur Unschädlichmachung von Mikroorganismen zu nutzen.

Gemäß einer Ausgestaltung ist der Sterilgut-Lagerungsbehälter zumindest abschnittsweise aus einem organischen Polymer gebildet. Bevorzugt, aber nicht notwendigerweise, ist das organische Polymer biologisch abbaubar. Damit kann vorteilhaft erreicht werden, dass auf erdölbasierte Kunststoffe verzichtet werden kann. Durch eine biologische Abbaubarkeit kann der Sterilgut-Lagerungsbehälter weiterhin vorteilhaft durch mikrobielle Zersetzung abgebaut werden und muss nicht verbrannt werden.

Bei dem organischen Polymer kann es sich beispielsweise um Zellulose oder Lignin aufweisende Polymere handeln. Es sind jedoch auch andere Polymere denkbar, welche eine für die vorgesehene Verwendung taugliche mechanische Stabilität des Sterilgut-Lagerungsbehälters erlauben.

Hierin wird unter einem organischen Polymer jede makromolekulare Verbindung verstanden, welche prinzipiell durch mikrobiologische Prozesse aufgespalten und abgebaut werden kann. Insoweit sind erfindungsgemäß auch andere Polymere als Zellulose oder Lignin erfasst.

Gemäß einer Ausgestaltung ist das organische Polymer mit einer funktionalen Beschichtung versehen, wobei diese dazu eingerichtet ist, das organische Polymer vor Feuchtigkeit zu schützen.

Durch die funktionelle Beschichtung kann vorteilhaft erreicht werden, dass ein zumindest abschnittsweise aus einem organischen Polymer gebildeter Sterilgut-Lagerungsbehälter chemisch stabiler und damit länger haltbar ist.

Bevorzugt ist die funktionale Beschichtung weiterhin dazu eingerichtet, dass ein zumindest abschnittsweise aus einem organischen Polymer gebildeter Sterilgut-Lagerungsbehälter mehrfach durch zumindest ein der oben genannten Sterilisationsverfahren sterilisierbar ist. Die funktionale Beschichtung trägt somit vorteilhaft zur chemischen Stabilität des Sterilgut-Lagerungsbehälters bei.

Funktionale Beschichtungen sind dem Fachmann aus dem Stand der Technik bekannt. Bspw. kann eine funktionale Beschichtung aus einem Polytetrafluorethylen gebildet sein, wobei die Schichtdicke frei ausgewählt werden kann.

Gemäß einer Ausführungsform weist die Sterilgut-Ausgabevorrichtung zumindest abschnittsweise eine fotokatalytische Beschichtung auf, wobei diese dazu eingerichtet ist, in Wirkverbundenheit mit der elektromagnetischen Welle eine Oberfläche der Sterilgut-Ausgabevorrichtung zu sterilisieren. Bevorzugt ist die fotokatalytische Beschichtung aus Titandioxid oder einem anderen dafür geeigneten Material gebildet.

Fotokatalytische Beschichtungen ermöglichen eine Selbstreinigung von damit behandelten Oberflächen, wobei diese mit Fotokatalysatoren, zum Beispiel Nanopartikeln aus Titandioxid (TiO2), beschichtet werden. Durch Bestrahlung mit hierin benannten elektromagnetischen Wellen, wie bspw. Sonnenlicht, werden organische Materialien (einschließlich mikrobieller Proteine) auf der behandelten Oberfläche zersetzt. Die Oberflächen bleiben sauber und wirken antimikrobiell.

Die photokatalytische Beschichtung ist dazu eingerichtet, Mikroorganismen im Wachstum zu hemmen oder abzutöten. Solche Beschichtungen sind dem Fachmann bekannt. Bspw. entwickelt das Fraunhofer IGB eine photokatalytisch aktive Beschichtung auf Basis von Titandioxid mit selbstreinigenden und selbstdesinfizierenden Eigenschaften. Die Bestrahlung dieser Schicht führt zur Bildung von freien Radikalen, welche Mikroorganismen abtöten. Solcherlei funktionelle Beschichtungen ermöglichen vorteilhaft eine antimikrobielle Wirkung ohne Einsatz biozider oder anderer chemischer Mittel. Bevorzugt ist die funktionelle Beschichtung im Bereich um die Ausgabeöffnung angeordnet. Insbesondere kann eine unterhalb des Ausgabemoduls angeordnete Schale mit einer solchen funktionellen Beschichtung ausgestaltet sein.

Wie oben ausgeführt wird unter einer Sterilisation hierin eine Verringerung der Keimzahl und/oder ein Erreichen eines deutlich reduzierten bis gar kein Keimwachstum bezeichnet.

Gemäß einer Ausführungsform weist der Sterilgut-Lagerungsbehälter einen Luftfilter auf. Ein Luftfilter ermöglicht vorteilhaft einen Gasaustausch zwischen dem Inneren des Sterilgut-Lagerungsbehälters und der Umgebung. Durch den Luftfilter wird weiterhin vorteilhaft die Sterilisation von innerhalb des Sterilgut-Lagerungsbehälters gelagertem Sterilgut verbessert. Bspw. kann während einer Dampfsterilisation in einem Autoklav der Dampf durch den Luftfilter in das Behälterinnere des Sterilgut-Lagerungsbehälters hineingelangen.

Gemäß einer Ausführungsform weist der Sterilgut-Lagerungsbehälter und/oder das untere Ausgabemodul zumindest ein transparentes Füllstandbeurteilungsmittel und/oder zumindest einen Füllstandsensor aufweist, wobei das transparente Füllstandbeurteilungsmittel und/oder der Füllstandsensor zu einer Beurteilung eines Füllstands des Sterilgut-Lagerungsbehälters eingerichtet ist.

In einer bevorzugten Ausgestaltung ist ein Füllstand des Sterilgut-Lagerungsbehälters, insbesondere ein Füllstand des Fassungsvolumens des Sterilgut-Lagerungsbehälters, durch ein Füllstandbeurteilungsmittel ermittelbar. Da der Sterilgut-Lagerungsbehälter sterilisierbar sein soll, ist es vorteilhaft, wenn das zum Ermitteln des Füllstandes erforderliche Mittel selbst auch sterilisierbar ist, sofern dieses von dem sterilisierbaren Sterilgut-Lagerungsbehälter umfasst ist. So kann es sich bei diesem Mittel um zumindest ein transparentes Füllstandbeurteilungsmittel (z.B. Sichtfenster) handeln. Dieses kann bspw. aus Glas oder einem Kunststoff gebildet sein, welcher mehrfach zumindest einem der oben genannten Sterilisationsverfahren unterworfen werden kann.

Es kann aber auch vorgesehen sein, dass das zum Ermitteln des Füllstandes zumindest ein Füllstandsensor zum Einsatz kommt. Dieser kann vom unteren Ausgabemodul umfasst sein und derart ausgestaltet sein, dass der Füllstandsensor ermittelt, welche Anzahl an Sterilgütern bereits ausgegeben worden ist, wobei der Sterilgut-Lagerungsbehälter eine definierte Anzahl an Sterilgütern aufweist. Abhängig von der Ausgestaltung des Sterilgut-Lagerungsbehälters, insbesondere dessen änderbares Fassungsvolumen, kann die Anzahl an auszugebendem Sterilgut variieren. Es kann sich beim Füllstandsensor um eine Elektronik handeln, die registriert, welche Menge an Sterilgut bereits aus dem Sterilgut-Lagerungsbehälter ausgegeben worden ist. Dem Fachmann sind Füllstandsensoren bekannt, welche sich für die vorgesehene Bestimmung eignen.

In einer Ausführungsform weist der Sterilgut-Lagerungsbehälter eine Kennung (z.B. Strichcode, QR-Code, RFID-Kennung) auf, welche den vorgesehenen Inhalt bei voll beladenem Zustand des Sterilgut-Lagerungsbehälters an das untere Ausgabemodul übermittelt. Hierfür muss das untere Ausgabemodul eine entsprechende Vorrichtung (z.B. Scanner, RFID-Reader) aufweisen, welche vom Fachmann frei gewählt werden kann.

Vorteilhaft an der Verwendung eines Sterilgut-Lagerungsbehälter ist, dass dieser mehrfach einem der oben genannten Sterilisationsverfahren unterzogen werden kann, was bei der Verwendung einer Kennung wie bspw. einer vom Sterilgut-Lagerungsbehälter umfassten RFID-Kennung möglicherweise nicht gegeben ist, sofern elektronische Komponenten durch das Sterilisationsverfahren (bspw. durch Hitze oder Strahlung) beeinträchtigt werden. Bevorzugt sind die elektronischen Komponenten jedoch so ausgestaltet, dass sie mehrfach einem Sterilisationsverfahren unterzogen werden können.

Vorteilhaft an der Verwendung einer RFID-Kennung, oder einer ähnlich gearteten Kennung ist, dass diese neu programmiert werden kann, um die vorgesehene maximale Befüllung des Sterilgut-Lagerungsbehälters mit einem Sterilgut anzugeben.

Ein Strich- oder QR-Code kann auswechselbar an dem Sterilgut-Lagerungsbehälter angebracht sein. So kann vorteilhaft erreicht werden, dass ein Code einfach durch einen anderen Code ausgetauscht werden kann.

In einer bevorzugten Ausgestaltung ist eine optische und/oder eine elektronische Benachrichtigung über den Füllstand des Sterilgut-Lagerungsbehälters erzeugbar. Unter einer optischen Benachrichtigung wird eine solche verstanden, welche ein optisches Signal (z.B. eine Emission von Licht) ausgibt. So ist denkbar, dass eine grüne LED leuchtet, solange der Sterilgut-Lagerungsbehälter bis zu 50 % voll ist und eine gelbe LED leuchtet, sobald ein Füllstand von 30 % erreicht ist und eine rote LED leuchtet, sobald der Sterilgut-Lagerungsbehälter einen Füllstand von weniger als 20 % erreicht hat oder leer ist. Ebenso kann ein Display, welches bspw. von dem unteren Ausgabemodul umfasst ist, dem Nutzer eine Auskunft über den Füllstand geben. Die möglichen Permutationen von Lichtfarbe oder Display-Anzeigen und korrespondierendem Füllstand können vom Fachmann frei gewählt werden.

Alternativ oder ergänzend dazu kann eine elektronische Benachrichtigung (bspw. über die oben beschriebenen elektrischen Kontakte) über den Füllstand des Sterilgut-Lagerungsbehälters an eine diese Information verarbeitende Stelle, bspw. ein Facility Management, erfolgen, so dass dieses über den Füllstand der Behälter (bevorzugt in Echtzeit) informiert ist und bei Bedarf den Sterilgut-Lagerungsbehälter austauschen kann. Weiterhin kann das Facility Management somit vorteilhaft die Auslastung von Sterilisationsanlagen mit neu zu beladenen und zu sterilisierenden oder neu zu bestellenden Sterilgut-Lagerungsbehältern (falls die Befüllung und die Sterilisation außer Haus erfolgt) besser planen.

Die vorliegende Offenbarung umfasst ferner auch ein hierin definiertes Suspensionsmittel zum magnetischen Suspendieren von Sterilgut, wobei das aus einem Sterilgut-Lagerungsbehälter ausgegebene Sterilgut durch ein Sterilgut-Suspensionsmodul magnetisch suspendiert wird. Das Suspensionsmittel ist für eine magnetische Wechselwirkung mit einem Sterilgut-Suspensionsmodul, eingerichtet, um das das Sterilgut magnetisch zu suspendieren. Vorzugsweise weist das Suspensionsmittel ein Metall auf, welches dazu eingerichtet ist von einem Magneten angezogen zu werden, welcher vom Sterilgut-Suspensionsmodul (hierin auch: Suspensionsmodul) umfasst ist.

Das Suspensionsmittel ist bevorzugt an einer das Sterilgut umgrenzenden Aufbewahrungshülle/Verpackung oder am Sterilgut selbst angeordnet. Das Suspensionsmittel ist, wie hierin beschrieben, für eine magnetische Wechselwirkung mit dem Sterilgut-Suspensionsmodul eingerichtet. Durch diese magnetische Wechselwirkung kann das Sterilgut bis zur Entnahme durch einen Nutzer von dem Sterilgut-Suspensionsmodul gehalten werden.

Die Aufgabe des Suspensionsmittels besteht darin, ein Mittel bereitzustellen, welches ermöglicht, dass ein bereits ausgegebenes Sterilgut bis zu dessen Entnahme aus der Vorrichtung durch einen Nutzer eine möglichst geringe oder bevorzugt gar keine Kontaktfläche zu einer (nicht sterilen) Oberfläche hat, so dass die Gefahr einer möglichen Kontamination des ausgegebenen Sterilguts minimiert ist.

Ein hierin beschriebenes Gut unterscheidet sich von einem Sterilgut lediglich dadurch, dass es nicht sterilisiert wird, oder nicht steril in einem Sterilgut-Lagerungsbehälter aufbewahrt wird oder aufbewahrt werden muss. Insoweit sind sämtliche hierin für das Sterilgut beschriebenen Ausführungsformen der erfindungsgemäßen Vorrichtung auch auf ein Gut anzuwenden. D.h., dass es sich nicht um einen Sterilgut-Lagerungsbehälter handelt, sofern darin ein Gut aufbewahrt werden soll, sondern um einen Gutlagerungsbehälter. Gleiches gilt sinngemäß für die anderen Begrifflichkeiten (Gut-Suspensionsmodul statt Sterilgut-Suspensionsmodul, etc.).

Weiterhin betrifft die Offenbarung ein Verfahren zum magnetischen Suspendieren eines Sterilguts innerhalb eines Sterilgut-Suspensionsmoduls, welches einen Magneten aufweist, wobei das Verfahren die folgenden Schritte umfasst:
a) Es wird ein Sterilgut-Lagerungsbehälter bereitgestellt,
b) Es wird ein Sterilgut bereitgestellt, wobei das Sterilgut ein weiter oben beschriebenes Suspensionsmittel aufweist,
c) Es wird ein Sterilgut-Suspensionsmodul bereitgestellt,
d) Es wird ein Sterilgut aus dem Sterilgut-Lagerungsbehälter ausgegeben, insbesondere berührungslos ausgegeben.

Das Sterilgut wird durch eine magnetische Wechselwirkung des Suspensionsmittels mit dem Sterilgut-Suspensionsmodul, bis zur Entnahme des Sterilguts aus dem Sterilgut-Suspensionsmodul magnetisch suspendiert.

Aufgabe der des Verfahren ist es, dass ein ausgegebenes Sterilgut bis zu dessen Entnahme durch einen Nutzer eine möglichst geringe, bevorzugt gar keine, Kontaktfläche zu einer (nicht sterilen) Oberfläche hat, so dass die Gefahr einer möglichen Kontamination des ausgegebenen Sterilguts minimiert wird.

Abschließend sei angemerkt, dass sämtlichen Merkmalen, die in den Anmeldungsunterlagen und insbesondere in den abhängigen Ansprüchen genannt sind, trotz des vorgenommenen formalen Rückbezugs auf einen oder mehrere bestimmte Ansprüche, auch einzeln oder in beliebiger Kombination eigenständiger Schutz zukommen soll.

Weitere Vorteile, Merkmale und Anwendungsmöglichkeiten der vorliegenden Erfindung ergeben sich auch aus der nachfolgenden Beschreibung von Ausführungsbeispielen und den Zeichnungen. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den Ansprüchen oder deren Rückbezügen. Zudem ist darauf hinzuweisen, dass der Fachmann zweifelsohne erkennt, dass sich die einzelnen Merkmale, die in den vorstehenden konkreten Ausführungsformen beschrieben sind, auf angemessene Weise miteinander kombinieren lassen, soweit kein Widerspruch vorliegt, wobei zum Vermeiden unnötiger Wiederholung auf eine separate Beschreibung verschiedener möglicher Kombinationen verzichtet wird.

### AUSFÜHRUNGSBEISPIELE

Nachfolgend wird die vorliegende Erfindung anhand von Ausführungsbeispielen unter Bezugnahme auf die beiliegenden Zeichnungen näher beschrieben, ohne die Erfindung auf diese zu beschränken.

Dabei zeigt
- **Fig. 1:**: Sterilgut-Ausgabevorrichtung (1.0) deren Fassungsvolumen (3.1) an die aufzunehmende Menge des Sterilgutes (2.0) angepasst werden kann
- **Fig. 2:**: Sterilgut-Ausgabevorrichtung (1.0) mit Sterilgut-Suspensionsmodul (7.0) und darin suspendiertes Sterilgut (2.0) im suspendierten Zustand A und herausgehobenen Zustand B
- **Fig. 3A:**: Seitenansicht der Sterilgut-Ausgabevorrichtung (1.0) mit Sterilgut-Lagerungsbehälter (3.0), der auf das untere Ausgabemodul (4.0) aufgeschoben wird, wobei die obere Abdeckvorrichtung (4.2) in das Gehäuse (4.3) geschoben wird
- **Fig. 3B:**: Frontalansicht der Sterilgut-Ausgabevorrichtung (1.0) mit Schienensystem (5.0) zur reversiblen Verbindung des Sterilgut-Lagerungsbehälters (3.0) mit dem unteren Ausgabemodul (4.0)
- **Fig. 4:**: Sterilgut (2.0) mit von diesem umfassten Suspensionsmittel (7.3) und magnetischem Halterungselement (7.2)
- **Fig. 5:**: Sterilgut-Ausgabevorrichtung (1.0) mit um ein Gelenk (4.4) drehbarem unteren Ausgabemodul (4.0)

In den unterschiedlichen Figuren sind hinsichtlich ihrer Funktion gleichwertige Teile stets mit denselben Bezugszeichen versehen, sodass diese in der Regel auch nur einmal beschrieben werden.

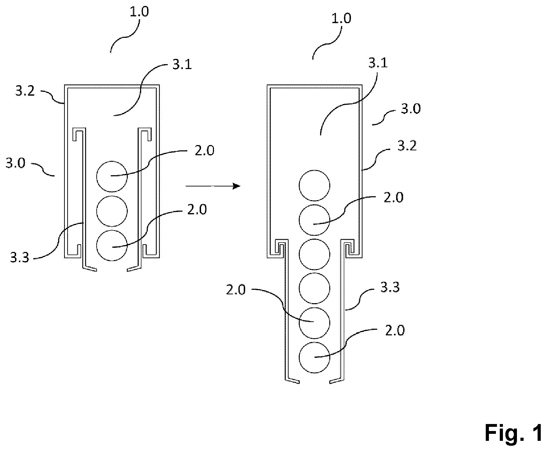

In Fig. 1 ist ein Abschnitt einer Ausführungsform einer Sterilgut-Ausgabevorrichtung (1.0), aufweisend einen Sterilgut-Lagerungsbehälter (3.0), gezeigt, bei der das Fassungsvolumen (3.1) des Sterilgut-Lagerungsbehälters (3.0) veränderlich ist. Prinzipiell umfasst der Sterilgut-Lagerungsbehälter (3.0) in diesem Ausführungsbeispiel zwei gegeneinander verschiebbare Behälterhälften (3.2, 3.3). Durch ein Bewegen der Behälterhälften (3.2, 3.3) relativ zueinander kann das Fassungsvolumen (3.1) an die Menge des aufzunehmenden Sterilgutes (2.0) angepasst werden. Die relative Position der Behälterhälften (3.2, 3.3) zueinander kann bspw. über einen Klemmmechanismus fixiert werden, woraufhin der Sterilgut-Lagerungsbehälter (3.0) mit dem Sterilgut (2.0) befüllt und sterilisiert werden kann. Aus Gründen einer einfachen Darstellung zeigt die Darstellung kein unteres Ausgabemodul.

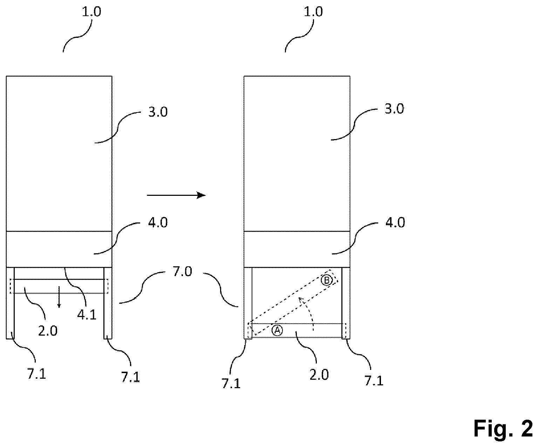

Die Fig. 2 zeigt eine Ausführungsform der Sterilgut-Ausgabevorrichtung (1.0), bei der das ausgegebene Sterilgut (2.0) von einem Sterilgut-Suspensionsmodul (7.0) aufgenommen/gehalten wird, nachdem das Sterilgut (2.0) die Ausgabeöffnung (4.1) verlassen hat. Hier ist das Sterilgut-Suspensionsmodul (7.0) im Wesentlichen aus zwei Halterungsschienen/Suspensionsschienen (7.1) gebildet, in welche das Sterilgut (2.0) hineinfallen/-gleiten kann. Das Sterilgut (2.0) kann dann vom Nutzer einfach aus den Halterungsschienen (7.1) gehoben werden. Die Position des hineingefallenen Sterilguts (2.0) ist mit "A" gekennzeichnet. Das Sterilgut (2.0) kann in der Position "B" aus den Halterungsschienen/Suspensionsschienen (7.1) gehoben werden.

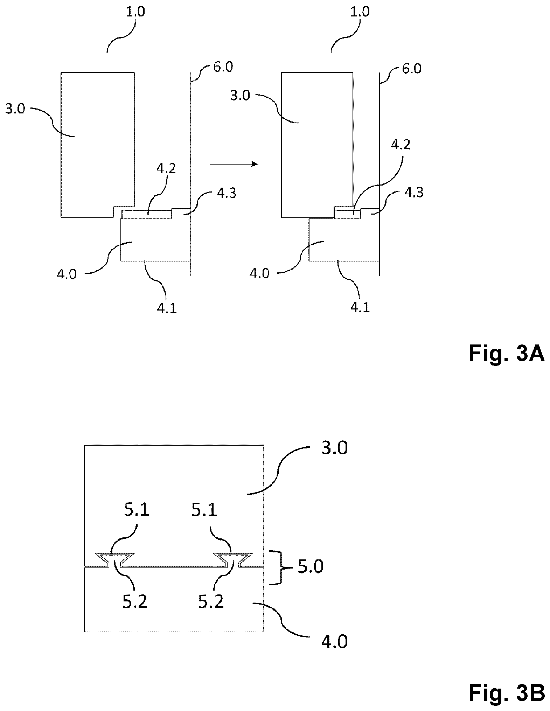

Die Fig. 3A zeigt eine Ausführungsform der Sterilgut-Ausgabevorrichtung (1.0), bei der der Sterilgut-Lagerungsbehälter (3.0) über ein Schienensystem (5.0) mit dem an einer Wand (6.0) befestigten unteren Ausgabemodul (4.0) verbunden werden kann. Das untere Ausgabemodul (4.0) weist an der Oberseite eine Abdeckvorrichtung (4.2) auf. Das in Fig. 3B dargestellte Schienensystem (5.0) umfasst im Wesentlichen Schienen (5.1, 5.2), die an der Oberseite des Ausgabemoduls (4.0) und an der Unterseite des Sterilgut-Lagerungsbehälters (3.0) angeordnet sind, wobei die Schienen (5.1, 5.2) gemäß einem Nut-Feder-Prinzip ineinandergreifen. Im Folgenden wird auf Fig. 3A Bezug genommen. Beim Aufschieben des Sterilgut-Lagerungsbehälters (3.0) auf das untere Ausgabemodul (4.0) wird die Abdeckvorrichtung (4.2) in das Gehäuse (4.3) geschoben. Damit kann erreicht werden, dass die Öffnung, durch welche das Sterilgut (2.0) aus dem Sterilgut-Lagerungsbehälter (3.0) in das untere Ausgabemodul (4.0) gelangt, abgedeckt ist, solange kein Sterilgut-Lagerungsbehälter (3.0) auf dem unteren Ausgabemodul (4.0) platziert ist. Um die Abdeckvorrichtung (4.2) wieder zu schließen, wenn der Sterilgut-Lagerungsbehälter (3.0) entnommen wird, umfasst das Gehäuse (4.3) ein nicht dargestelltes Rückstellsystem, bspw. unter Beteiligung von Federn. Der gleiche Mechanismus befindet sich auch an der Unterseite des Sterilgut-Lagerungsbehälters (3.0) (nicht gezeigt). Es ist auch denkbar, dass das untere Ausgabemodul (4.0) und der Sterilgut-Lagerungsbehälter (3.0) jeweils zumindest zwei Abdeckvorrichtungen (4.2) aufweisen, die zueinander versetzt sind. So könnte durch das Aufschieben des Sterilgut-Lagerungsbehälters (3.0) auf das Ausgabemodul (4.0) zuerst eine obere/äußere Abdeckvorrichtung (4.2) geöffnet werden. Ein weiteres Aufschieben würde dann eine von der oberen/äußeren Abdeckvorrichtung (4.2) abgedeckte untere/innere Abdeckvorrichtung (nicht dargestellt) freigeben, welche während des weiteren Aufschiebens geöffnet wird. Im Falle des Sterilgut-Lagerungsbehälters (3.0) ist mit einer oberen/äußeren Abdeckvorrichtung diejenige gemeint, welche die obere Abdeckvorrichtung (4.2) des unteren Ausgabemoduls (4.0) kontaktiert. Durch das Aufschieben des Sterilgut-Lagerungsbehälters (3.0) auf das untere Ausgabemodul (4.0), insbesondere der Bewegung der oberen/äußeren Abdeckvorrichtung gegen eine dieser gegenüberliegenden Abdeckvorrichtung, kann ein Mechanismus aktiviert werden, welcher dafür sorgt, dass die untere/innere Abdeckvorrichtung hin zu einer dieser gegenüberliegenden inneren Abdeckvorrichtung bewegt wird, um diese zu kontaktieren und zu öffnen.

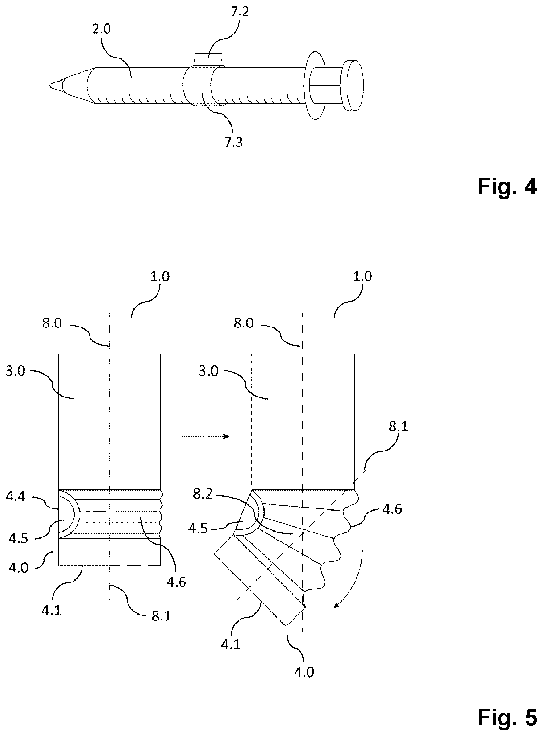

In **Fig. 4** ist ein Sterilgut (2.0) gezeigt, welches einen als Metallring ausgebildetes Suspensionsmittel (7.3) aufweist. Dieses ist dazu eingerichtet, um von einem vom Sterilgut-Suspensionsmodul (7.0) umfassten magnetischen Halterungselement (7.2) aufgrund einer magnetischen Wechselwirkung angezogen zu werden, so dass das Sterilgut (2.0) von diesem (kraftschlüssig) gehalten werden kann. Das magnetische Halterungselement (7.2) ist hier als ein Permanentmagnet ausgebildet, welcher an der Unterseite der Sterilgut-Ausgabevorrichtung (1.0) (nicht gezeigt) angeordnet ist. Weiterhin oder alternativ kann das magnetische Halterungselement (7.2) einen Elektromagneten aufweisen, welcher durch eine Elektronik so angesteuert werden kann, dass das Sterilgut (2.0) nach Ausgabe aus dem Sterilgut-Lagerungsbehälter (3.0) bis zur Entnahme nur einen Nutzer in der Schwebe gehalten wird.

In der in **Fig. 5** dargestellten Ausführungsform ist ein Winkel (8.2) zwischen der Mittelachse (8.1) der Ausgabeöffnung (4.1) und der Längsachse (8.0) des Sterilgut-Lagerungsbehälters (3.0) einstellbar. Um die Einstellbarkeit zu erreichen, umfasst das untere Ausgabemodul (4.0) ein Gelenk (4.4), um welches das Ausgabemodul (4.0) bewegbar ist. Vorzugsweise kann durch ein Drücken eines am Gelenk (4.4) angeordneten Tasters (4.5) das Gelenk neu ausgerichtet werden, bis es an der gewünschten Position einrastet. Zwischen der Ausgabeöffnung (4.1) und dem unteren Bereich des Sterilgut-Lagerungsbehälters (3.0) ist ein Umlenkschlauch (4.6) angeordnet. Bei diesem handelt es sich im Wesentlichen um ein flexibles Element, welches nicht von Mikroorganismen, aber bevorzugt von Gasen passiert werden kann. Durch die Bewegung des Gelenks (4.4) dahingehend, dass der Winkel (8.2) verkleinert wird, kommt es zu einer Dehnung des dem Gelenk (4.4) gegenüberliegenden Bereiches des Umlenkschlauches (4.6). Ein den Sterilgut-Lagerungsbehälter (3.0) verlassendes Sterilgut (2.0) passiert zuerst den Umlenkschlauch (4.6), welcher einen Teil der kinetischen Energie des Sterilgutes (2.0) aufnimmt, bevor das Sterilgut (2.0) zur Ausgabeöffnung (4.1) gelangt, so dass das Herausfallen des Sterilguts (2.0) kontrollierter abläuft.

### BEZUGSZEICHENLISTE

- 1.0: Sterilgut-Ausgabevorrichtung
- 2.0: Sterilgut
- 3.0: Sterilgut-Lagerungsbehälter
- 3.1: Fassungsvolumen
- 3.2: erste/obere Behälterhälfte
- 3.3: zweite/untere Behälterhälfte
- 4.0: (unteres) Ausgabemodul
- 4.1: Ausgabeöffnung
- 4.2: (obere/äußere) Abdeckvorrichtung
- 4.3: Gehäuse
- 4.4: Gelenk
- 4.5: Taster
- 4.6: Umlenkschlauch
- 5.0: Schienensystem
- 5.1: Nut-Schiene
- 5.2: Feder-Schiene
- 6.0: Wand
- 7.0: Sterilgut-Suspensionsmodul
- 7.1: Halterungsschiene/Suspensionsschiene
- 7.2: magnetisches Halterungselement
- 7.3: Suspensionsmittel
- 8.0: Längsachse
- 8.1: Mittelachse
- 8.2: Winkel

## Patentansprüche

1. Sterilgut-Ausgabevorrichtung (1.0) zum Lagern und Ausgeben von Sterilgut (2.0), aufweisend einen Sterilgut-Lagerungsbehälter (3.0) sowie ein unteres Ausgabemodul (4.0),
wobei der Sterilgut-Lagerungsbehälter (3.0) für eine sterile Lagerung des Sterilguts (2.0) eingerichtet ist,
wobei das untere Ausgabemodul (4.0) eine Ausgabeöffnung (4.1) aufweist, die zur Ausgabe von Sterilgut (2.0) eingerichtet ist,
wobei der Sterilgut-Lagerungsbehälter (3.0) dazu eingerichtet ist, mit dem Sterilgut (2.0) befüllt zu werden,
wobei der Sterilgut-Lagerungsbehälter (3.0) dazu eingerichtet ist, mit oder ohne innerhalb des Sterilgut-Lagerungsbehälters (3.0) gelagertem Sterilgut (2.0) sterilisiert zu werden,
wobei die Sterilgut-Ausgabevorrichtung (1.0) zur berührungslosen Ausgabe von Sterilgut (2.0) aus dem Sterilgut-Lagerungsbehälter (3.0) eingerichtet ist,
der Sterilgut-Lagerungsbehälter (3.0) und das untere Ausgabemodul (4.0) dazu eingerichtet sind reversibel miteinander verbunden zu werden, wobei das Sterilgut (2.0) erst nach seiner Ausgabe durch das untere Ausgabemodul (4.0) einer nichtsterilen Umgebung exponiert ist,
**dadurch gekennzeichnet, dass**
das untere Ausgabemodul (4.0) ein Sterilgut-Suspensionsmodul (7.0) aufweist, welches dazu eingerichtet ist, das ausgegebene Sterilgut (2.0) bis zu dessen Entnahme durch einen Nutzer mechanisch oder magnetisch zu suspendieren.

2. Sterilgut-Ausgabevorrichtung gemäß Anspruch 1, wobei der Sterilgut-Lagerungsbehälter (3.0) aus zumindest zwei Behälterhälften (3.2, 3.3) gebildet ist, wobei die Behälterhälften (3.2, 3.3) dazu eingerichtet sind, gegeneinander verschoben zu werden.

3. Sterilgut-Ausgabevorrichtung gemäß Anspruch 1 oder 2, wobei die Sterilgut-Ausgabevorrichtung (1.0) ein Ausgabemodul-Halterungsmittel aufweist, welches für eine reversible Verbindung mit dem unteren Ausgabemodul (4.0) eingerichtet ist, wobei das Ausgabemodul-Halterungsmittel einen ersten elektrischen Kontaktpunkt und das untere Ausgabemodul (4.0) einen zweiten elektrischen Kontaktpunkt aufweist.

4. Sterilgut-Ausgabevorrichtung gemäß Anspruch 3, wobei eine erfolgte reversible Verbindung dazu eingerichtet ist den ersten elektrischen Kontaktpunkt mit dem zweiten elektrischen Kontaktpunkt zu kontaktieren.

5. Sterilgut-Ausgabevorrichtung gemäß einem der Ansprüche 1 bis **4,** wobei das untere Ausgabemodul (4.0) zumindest zwei mechanische Kontaktpunkte aufweist, wobei diese für eine mechanische Suspension von ausgegebenem Sterilgut (2.0) eingerichtet sind.

6. Sterilgut-Ausgabevorrichtung gemäß einem der Ansprüche 1 bis **4,** wobei das Sterilgut-Suspensionsmodul (7.0) zumindest eine Suspensionsschiene aufweist, wobei diese für eine mechanische Suspension von ausgegebenem Sterilgut (2.0) eingerichtet ist.

7. Sterilgut-Ausgabevorrichtung gemäß einem der Ansprüche 1 bis **4,** wobei das Sterilgut-Suspensionsmodul (7.0) ein magnetisches Suspensionselement (7.2) aufweist, welches dazu eingerichtet ist das ausgegebene Sterilgut (2.0) bis zu dessen Entnahme durch einen Nutzer magnetisch zu suspendieren oder in einer magnetischen Schwebe zu halten.

8. Sterilgut-Ausgabevorrichtung gemäß einem der Ansprüche 1 bis 7, wobei die Sterilgut-Ausgabevorrichtung (1.0), insbesondere das untere Ausgabemodul (4.0), und/oder eine Wandvorrichtung ein Sterilisationsmodul aufweist, wobei das Sterilisationsmodul zu einer Emission einer elektromagnetischen Welle und dazu eingerichtet ist, das vom Sterilgut-Suspensionsmodul (7.0) mechanisch oder magnetisch suspendierte Sterilgut (2.0) mit einer elektromagnetischen Welle zu bestrahlen.

9. Sterilgut-Ausgabevorrichtung gemäß einem der Ansprüche 1 bis 8, wobei der Sterilgut-Lagerungsbehälter (3.0) zumindest abschnittsweise aus einem organischen Polymer gebildet ist.

10. Sterilgut-Ausgabevorrichtung gemäß Anspruch 9, wobei das organische Polymer mit einer funktionalen Beschichtung versehen ist, wobei diese dazu eingerichtet ist, das organische Polymer vor Feuchtigkeit zu schützen.

11. Sterilgut-Ausgabevorrichtung gemäß einem der Ansprüche 8 bis 10, wobei die Sterilgut-Ausgabevorrichtung (1.0) zumindest abschnittsweise eine fotokatalytische Beschichtung aufweist, wobei diese dazu eingerichtet ist, in Wirkverbundenheit mit der elektromagnetischen Welle eine Oberfläche der Sterilgut-Ausgabevorrichtung (1.0) zu sterilisieren.

12. Sterilgut-Ausgabevorrichtung gemäß einem der Ansprüche 1 bis 11, wobei der Sterilgut-Lagerungsbehälter (3.0) einen Luftfilter aufweist.

## Claims

1. **Sterile goods dispensing device** (1.0) for storing and dispensing sterile goods (2.0), comprising a sterile goods storage container (3.0) and a lower dispensing module (4.0),
wherein the sterile goods storage container (3.0) is configured for sterile storage of the sterile goods (2.0),
wherein the lower dispensing module (4.0) comprises a dispensing opening (4.1) configured for dispensing sterile goods (2.0),
wherein the sterile goods storage container (3.0) is configured to be filled with the sterile goods (2.0),
wherein the sterile goods storage container (3.0) is configured to be sterilized with or without sterile goods (2.0) stored inside the sterile goods storage container (3.0),
wherein the sterile goods dispensing device (1.0) is configured for the contactless dispensing of sterile goods (2.0) from the sterile goods storage container (3.0),
the sterile goods storage container (3.0) and the lower dispensing module (4.0) are configured to be reversibly connected to one another, wherein the sterile goods (2.0) are exposed to a non-sterile environment only after their dispensing by the lower dispensing module (4.0),
**characterized in that**
the lower dispensing module (4.0) comprises a sterile goods suspension module (7.0), which is configured to suspend the dispensed sterile goods (2.0) mechanically or magnetically until removal by a user.

2. Sterile goods dispensing device according to claim 1, wherein the sterile goods storage container (3.0) is formed from at least two container halves (3.2, 3.3), wherein the container halves (3.2, 3.3) are configured to be displaced relative to one another.

3. Sterile goods dispensing device according to claim 1 or 2, wherein the sterile goods dispensing device (1.0) comprises a dispensing module retaining means configured for reversible connection to the lower dispensing module (4.0), wherein the dispensing module retaining means has a first electrical contact point and the lower dispensing module (4.0) has a second electrical contact point.

4. Sterile goods dispensing device according to claim 3, wherein an established reversible connection is configured to contact the first electrical contact point with the second electrical contact point.

5. Sterile goods dispensing device according to any one of claims 1 to 4, wherein the lower dispensing module (4.0) has at least two mechanical contact points, wherein these are configured for mechanical suspension of dispensed sterile goods (2.0).

6. Sterile goods dispensing device according to any one of claims 1 to 4, wherein the sterile goods suspension module (7.0) has at least one suspension rail, which is configured for the mechanical suspension of dispensed sterile goods (2.0).

7. Sterile goods dispensing device according to any one of claims 1 through 4, wherein the sterile goods suspension module (7.0) has a magnetic suspension element (7.2) which is configured to magnetically suspend the dispensed sterile goods (2.0) or to hold it in a magnetic levitation until removal by a user.

8. Sterile goods dispensing device according to any one of claims 1 to 7, wherein the sterile goods dispensing device (1.0), in particular the lower dispensing module (4.0), and/or a wall unit comprises a sterilization module, wherein the sterilization module is configured to emit an electromagnetic wave and to irradiate the sterile goods (2.0) mechanically or magnetically suspended by the sterile goods suspension module (7.0) with an electromagnetic wave.

9. Sterile goods dispensing device according to any one of claims 1 to 8, wherein the sterile goods storage container (3.0) is formed at least in part from an organic polymer.

10. Sterile goods dispensing device according to claim 9, wherein the organic polymer is provided with a functional coating, which is configured to protect the organic polymer from moisture.

11. Sterile goods dispensing device according to any one of claims 8 to 10, wherein the sterile goods dispensing device (1.0) comprises, at least in sections, a photocatalytic coating, which is configured to sterilize a surface of the sterile goods dispensing device (1.0) in an operative connection with the electromagnetic wave.

12. Sterile goods dispensing device according to one of claims 1 to 11, wherein the sterile goods storage container (3.0) has an air filter.

## Revendications

1. Dispositif de distribution de produits stériles (1.0) pour stocker et distribuer des produits stériles (2.0), comportant un contenant de stockage (3.0) de produits stériles ainsi qu'un module de distribution inférieur (4.0),
le contenant de stockage (3.0) de produits stériles étant mis au point pour un stockage stérile des produits stériles (2.0),
le module de distribution inférieur (4.0) comportant une ouverture de distribution (4.1), qui est mise au point pour distribuer des produits stériles (2.0),
le contenant de stockage (3.0) de produits stériles étant mis au point pour être rempli avec les produits stériles (2.0),
le contenant de stockage (3.0) de produits stériles étant mis au point pour être stérilisé avec ou sans produits stériles (2.0) stockés à l'intérieur du contenant de stockage (3.0) de produits stériles,
le dispositif de distribution de produits stériles (1.0) étant mis au point pour distribuer sans contact des produits stériles (2.0) provenant du contenant de stockage (3.0) de produits stériles,
le contenant de stockage (3.0) de produits stériles et le module de distribution inférieur (4.0) étant mis au point pour être reliés l'un à l'autre de manière réversible, les produits stériles (2.0) n'étant exposés à un environnement non stérile qu'après avoir été distribués par le module de distribution inférieur (4.0), **caractérisé en ce que**
le module de distribution inférieur (4.0) comporte un module de suspension (7.0) de produits stériles, qui est mis au point pour suspendre mécaniquement ou magnétiquement les produits stériles (2.0) distribués jusqu'à ce qu'ils soient retirés par un utilisateur.

2. Dispositif de distribution de produits stériles selon la revendication 1, **caractérisé en ce que** le contenant de stockage (3.0) de produits stériles est formé à partir d'au moins deux moitiés de contenant (3.2, 3.3), les moitiés de contenant (3.2, 3.3) étant mises au point pour être coulissées l'une à l'encontre de l'autre.

3. Dispositif de distribution de produits stériles selon la revendication 1 ou 2, le dispositif de distribution (1.0) de produits stériles comportant un moyen de support de module de distribution, qui est mis au point pour une liaison réversible au module de distribution inférieur (4.0), le moyen de support de module de distribution comportant un premier point de contact électrique et le module de distribution inférieur (4.0) comportant un deuxième point de contact électrique.

4. Dispositif de distribution de produits stériles selon la revendication 3, une liaison réversible qui a eu lieu étant mise au point pour entrer en contact avec le premier point de contact électrique par le deuxième point de contact électrique.

5. Dispositif de distribution de produits stériles selon l'une des revendications 1 à 4, le module de distribution inférieur (4.0) comportant au moins deux points de contact mécaniques, ceux-ci étant mis au point pour une suspension mécanique de produits stériles (2.0) distribués.

6. Dispositif de distribution de produits stériles selon l'une des revendications 1 à 4, le module de suspension (7.0) de produits stériles comportant au moins un rail de suspension, ledit rail de suspension étant mis au point pour une suspension mécanique de produits stériles (2.0) distribués.

7. Dispositif de distribution de produits stériles selon l'une des revendications 1 à 4, le module de suspension (7.0) de produits stériles comportant un élément de suspension magnétique (7.2) qui est mis au point pour suspendre ou maintenir magnétiquement les produits stériles (2.0) distribués dans une suspension magnétique jusqu'à ce qu'ils soient retirés par un utilisateur.

8. Dispositif de distribution de produits stériles selon l'une des revendications 1 à 7, **caractérisé en ce que** le dispositif de distribution (1.0) de produits stériles, en particulier le module de distribution inférieur (4.0), et/ou un dispositif mural comportent un module de stérilisation, le module de stérilisation étant mis au point pour émettre une onde électromagnétique et pour irradier les produits stériles (2.0) suspendus mécaniquement ou magnétiquement par le module de suspension (7.0) de produits stériles avec une onde électromagnétique.

9. Dispositif de distribution de produits stériles selon l'une des revendications 1 à 8, le contenant de stockage (3.0) de produits stériles étant formé au moins par sections à partir d'un polymère organique.

10. Dispositif de distribution de produits stériles selon la revendication 9, le polymère organique étant pourvu d'un revêtement fonctionnel, ledit revêtement étant mis au point pour protéger le polymère organique de l'humidité.

11. Dispositif de distribution de produits stériles selon l'une des revendications 8 à 10, le dispositif de distribution (1.0) de produits stériles comportant un revêtement photocatalytique au moins par sections, ledit revêtement photocatalytique étant mis au point pour stériliser une surface du dispositif de distribution (1.0) de produits stériles en interaction fonctionnelle avec l'onde électromagnétique.

12. Dispositif de distribution de produits stériles selon l'une des revendications 1 à 11, le contenant de stockage (3.0) de produits stériles comportant un filtre à air.
